Europäisches Patentamt

(19) European Patent Office

Office européen des brevets

(11) **EP 0 803 573 A1**

(12) **EUROPEAN PATENT APPLICATION**

(43) Date of publication:
29.10.1997 Bulletin 1997/44

(21) Application number: 96106531.5

(22) Date of filing: 25.04.1996

(51) Int. Cl.[6]: **C12N 15/62**, C07K 14/005,
C07K 14/195, C07K 14/37,
C07K 14/44, C07K 14/47,
C07K 14/52, A61K 39/295

(84) Designated Contracting States:
AT BE CH DE DK ES FI FR GB GR IE IT LI LU MC
NL PT SE

(71) Applicant: **Gesellschaft für Biotechnologische Forschung mbH (GBF)**
**D-38124 Braunschweig (DE)**

(72) Inventors:
• **Hauser, Hansjörg, Dr.**
**D-38124 Braunschweig (DE)**

• **Wirth, Dagmar, Dr.**
**D-38124 Braunschweig (DE)**
• **Reimann, Jörg, Prof. Dr.**
**D-89069 Ulm (DE)**

(74) Representative: **Boeters, Hans Dietrich, Dr. et al**
**Patentanwälte Boeters & Bauer,**
**Bereiteranger 15**
**81541 München (DE)**

(54) **Polycistronic expression construct with cytokines for multivalent vaccines**

(57) **Summary**

The invention concerns a polycistronic expression construct of a vector comprising at least one antigen gene and at least one cytokine gene or comprising at least two antigen genes and, facultatively, at least one cytokine gene; a vaccine comprising a polycistronic expression construct according to the invention; and, in addition, their use.

EP 0 803 573 A1

## Description

## Background.

*Polycistronic expression constructs.* A 'foreign' polypeptide can be expressed in a vertebrate cell when an expression plasmid DNA that encodes the polypeptide gene cloned into the construct of the plasmid is introduced into the cell. Polycistronic expression plasmids allow the simultaneous expression of two and more distinct gene products at stochiometrically defined ratios from a single transcript within the transfected cell; ref. is made, for example, to WO-A1-9 405 785 and WO-A1-9 405 786.

*The adaptive immune system of vertebrates.* Two major compartments constitute the adaptive immune system of mammals: the humoral and the cellular system. Humoral immunity is mediated by soluble protein molecules (antibodies) secreted by a specialized class of lymphocytes (B cells) into body fluids. The variable (polymorphic) part of the antibody molecules binds directly to native and denatured antigens (of diverse chemical composition) in the soluble phase. In contrast, cellular immunity is mediated by a different class of lymphocytes (T cells) that recognize only cell-associated protein antigens that have been intracellularly processed (partially digested) and are presented on the cell surface of antigen presenting cells (APC) in the context of major histocompatibility complex (MHC) glycoproteins.

Specific, protective immune reactivity is generated when the right type of effector function is delivered in sufficient strength at the right time at the site of emergence of 'foreign' antigen in the organism. Antigen-recognizing T lymphocytes potentially express a large repertoire of effector molecules, such as for example cytokines. A particular antigen-stimulated clone expresses only a minor subset of effector molecules from the large potential repertoire, *i.e.*, only a limited set of effector functions is coexpressed in a particular T lymphocyte clone while most effector molecules are not produced. Particular functional phenotypes of T cells are hence clonally distributed. This known finding is of importance for vaccine designs because the preparation of an antigen and its mode of delivery critically influence the type of immune response it elicits. Depending on the mode of vaccination, the natural challenge of a vaccinated host with the respective pathogen may lead, either to stable protection, or to aggravation of disease and immunopathology. Today's major objective of vaccine design is to find antigens and ways to deliver them to induce stable, protective responses and to avoid harmful responses.

*The cellular immune system.* The T cell system is composed of two subsets that differ in surface marker expression and functional phenotype, as well as in the restricting class of MHC molecules that present antigenic peptides to the respective T cell subsets. $CD8^+$ 'killer' (cytotoxic) T lymphocytes (CTL) recognize antigen in the context of MHC class I molecules, are often cytotoxic, and express interferon-$\tau$. $CD4^+$ 'helper' T cells recognize antigen in the context of MHC class II molecules, are usually not cytotoxic, and express different, mutually exclusive profiles of cytokines.

Membrane glycoproteins encoded within the MHC control specific activation of T cells. T cells do not recognize native antigens but respond to peptide fragments of protein antigens presented on the surface of antigen presenting cells (APC) by polymorphic MHC molecules. Processing of protein antigens is required to specifically stimulate T cells. Different pathways of intracellular processing of protein antigens control the activation of $CD4^+$ and $CD8^+$ T cell responses. In the *exogenous processing pathway*, extracellular protein antigens are endocytosed by APC and partially degraded in a specialized endosomal compartment to 12 to 15 residue peptides by acid proteolysis. These peptides bind haplotype-specific to MHC class II molecules and subsequently transit to the surface of the APC. Soluble protein antigens processed in this pathway preferentially elicit MHC class II-restricted $CD4^+$ T cell responses. MHC class I-restricted $CD8^+$ CTL responses are stimulated by protein antigens processed in the alternate *endogenous processing pathway*. In this pathway, antigenec peptides derived from cytosolic proteins are transported into the lumen of the endoplasmic reticulum (ER) by a peptide transporter complex where they bind to nascent MHC class I heavy chain/b$_2$m microglobulin dimers. This generates trimeric, transport-competent MHC class I complexes that move rapidly by the default secretory route to the APC surface. Thus, protein antigens derived from, either an exogenous, or an endogenous source, seem to be processed in two alternative, mutually exclusive pathways for MHC restricted presentation of antigenic peptides to T cells.

*Design of recombinant subunit vaccines.* Vaccination with immunogenic, recombinant protein antigens can attenuate or eliminate disease manifestations induced by microbial pathogens. Various problems have emerged that limit the general usefulness of recombinant protein antigens as vaccines. Many recombinant proteins are of low intrinsic immunogenicity and require association with strong adjuvants to elicit an immune response. Given as soluble, native protein antigens, they usually do not prime many types of T cell responses. When mixtures of antigenic proteins are injected, the immune response against one of them (the 'immunodominant' antigen) often dominates the response.

Insight into the molecular basis of specific immune reactions, in particular T cell-mediated responses, has expanded dramatically. This has made the design of vaccines aimed at the preferential stimulation of a particular type of immune response possible. Most efficient are the currently available vaccines that eliminate extracellular pathogens or toxins through antibody (B cell)-mediated immune reactions. In contrast, less efficient are many vaccines against intracellular pathogens that rely on T cell-mediated immune reactivities. Vaccination is currently used as a prophylactic as well as a therapeutic technique. Designs of vaccines or immunotherapies have been of interest in the field of infectious disease, cancer research and autoimmune diseases. Hence, the spectrum of potential applications of vaccines has been considerably enlarged.

*DNA-based vaccination.* New approaches to vaccination are opened by the injection of plasmid DNA; cf., for example, WO-A1-9 011 092. For the design of DNA vaccines, the vector constructs used, their mode of delivery, and the spectrum of specific immune reactivities they elicit, are of interest. Intramuscular inoculation of plasmid DNA carrying microbial genes cloned under appropriate promoter control has been shown to prime specific immune responses and to confer protection against influenza virus, rabies virus, lymphocytic choriomenigitis virus induced pathology, malaria, or leishmaniasis. Intramuscular DNA vaccination seems attractive because of its efficacy. The inoculated plasmid DNA seems to persist episomally without replication in the nuclei of myocytes without integrating into the genome. Expression of antigens after intramuscular plasmid DNA injection has been shown in striated muscle cells. This may cause persistent antigen presentation leading to prolonged stimulation of the immune response. DNA-based vaccination efficiently induces MHC class I-restricted cytotoxic T lymphocyte (CTL) responses and serum antibody responses to different antigens.

Up to now polycistronic expression constructs have, however, been used merely in vitro. Further, polycistronic expression constructs have not yet been suggested for any use for an DNA-based vaccination. An approach for the use of multi-gene vectors for vaccination has, however, been described in Genetic Engineering News, (March 1) (1996) 14-16.

According to a first embodiment the problem underlaying the invention is solved by a polycistronic expression construct of a vector comprising at least one antigen gene and at least one cytokine gene.

According to a second embodiment the problem underlaying the invention is solved by a polycistronic expression construct of a vector comprising at least two antigen genes and, facultatively, at least one cytokine gene.

A preferred embodiment concerns an expression construct according to the invention, which is characterized in that the antigen genes are viral, bacterial, fungal, protozoal, tumor and/or prion antigens.

Another preferred embodiment concerns an expression construct according to the invention, which is characterized in that the antigen genes are from the same and/or different pathogens.

Another preferred embodiment concerns an expression construct according to the invention, which is characterized by one or more genes coding for chimeric proteins composed of immunogenic domains of different proteins.

Another preferred embodiment concerns an expression construct according to the invention, which is characterized in that the antigen genes are derived from small viruses, especially pathogenic strains of hepadna, papova, and/or retro viruses.

Another preferred embodiment concerns an expression construct according to the invention, which is characterized by cAg, sAg, LS, HBVpol L1 and/or cT-Ag as viral antigens.

Another preferred embodiment concerns an expression construct according to the invention, which is characterized by cytokine genes encoding IL, especially IL-2, IL-4, IL-6, IL-7, IL-10, IL-12 and/or IL-15; IFN, especially IFNalpha, IFNβ and/or IFNg; and/or GM-CSF.

Another preferred embodiment concerns an expression construct according to the invention, which is characterized in that it comprises more than one and especially two or more than two genes of the same cytokine.

Another preferred embodiment concerns an expression construct according to the invention, which comprises the following genes:

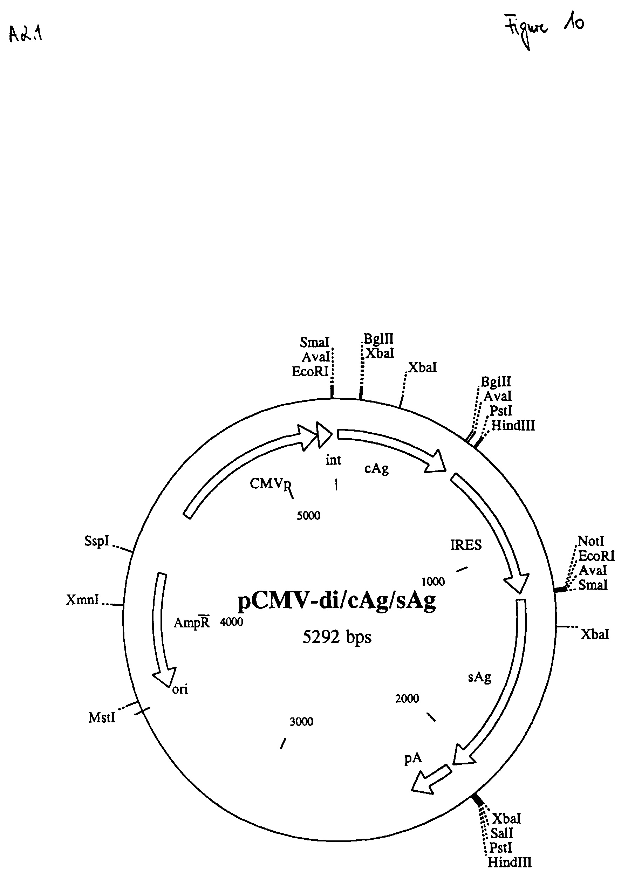

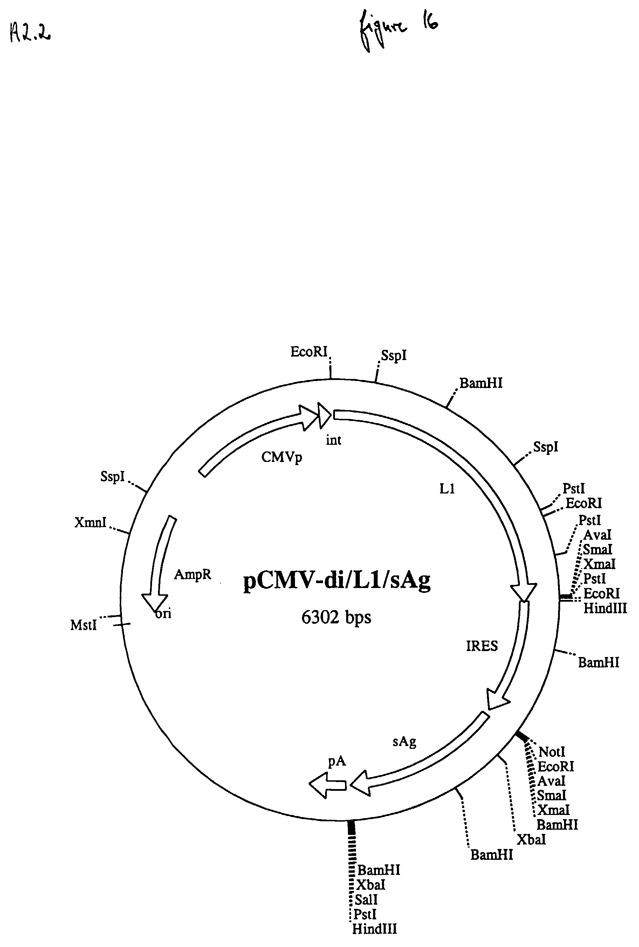

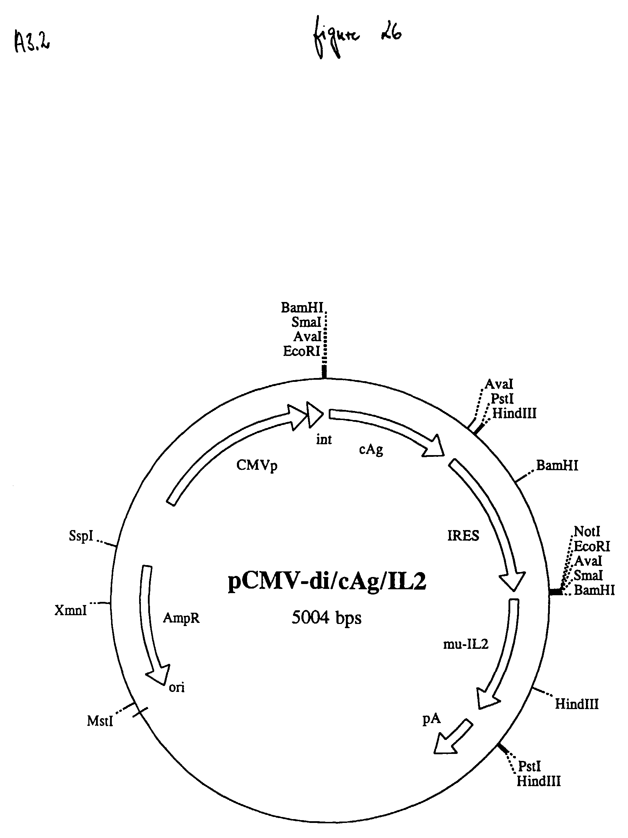

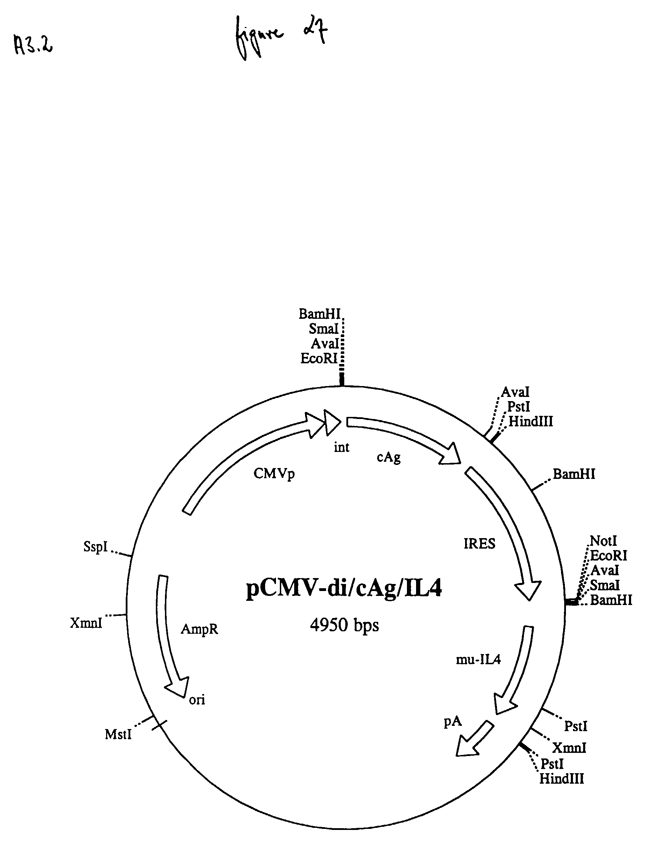

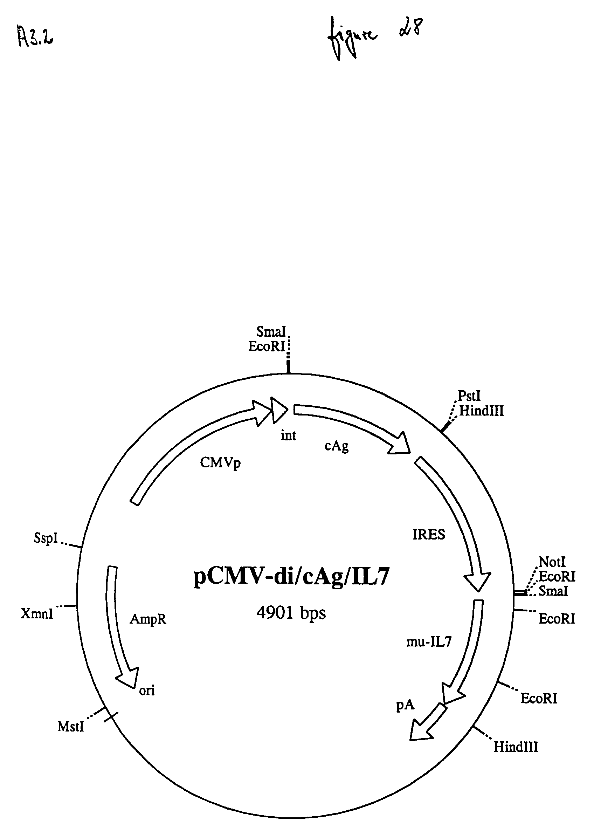

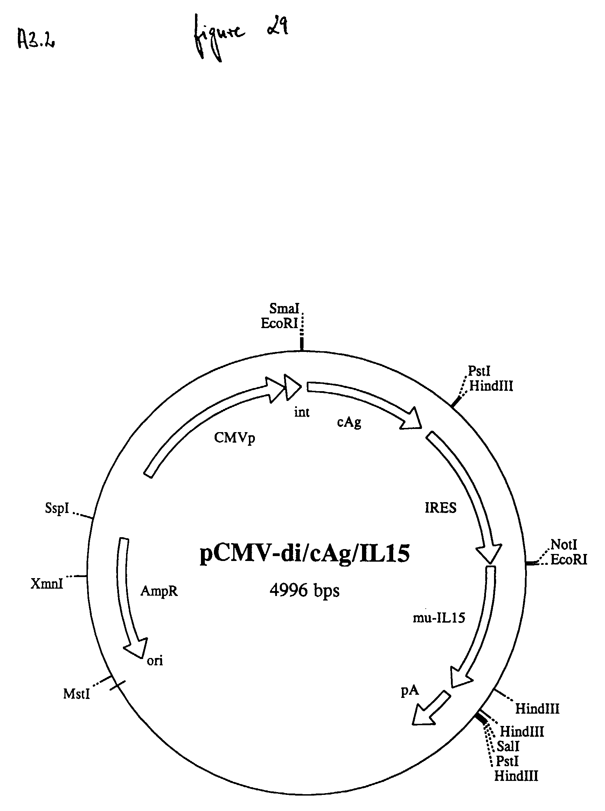

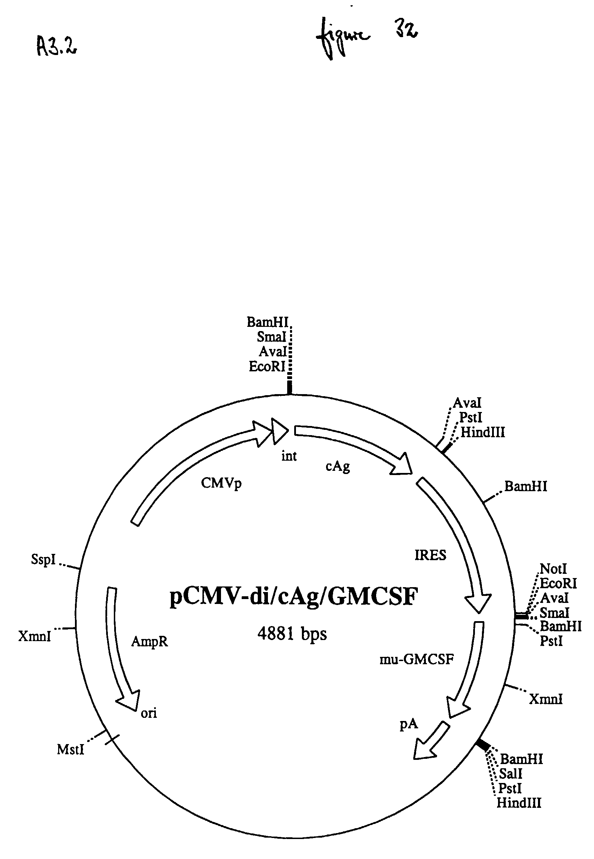

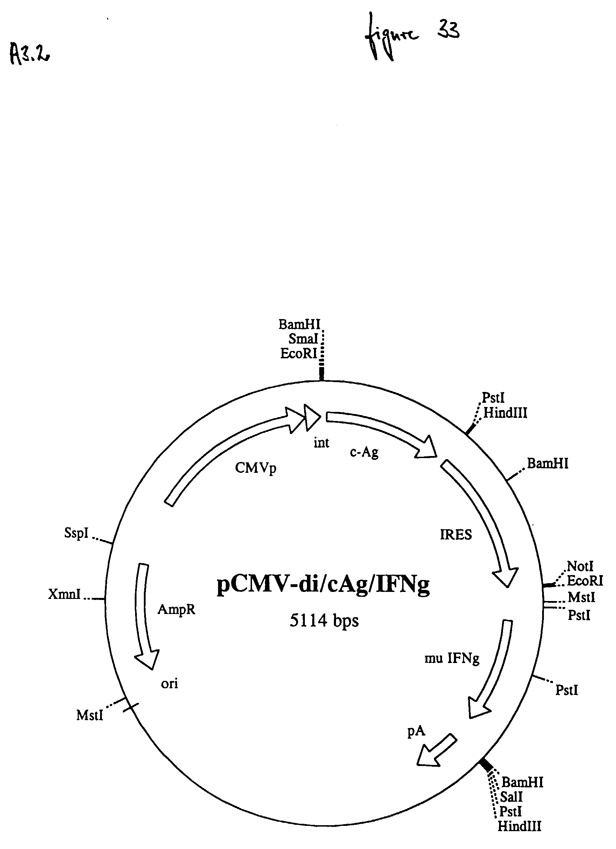

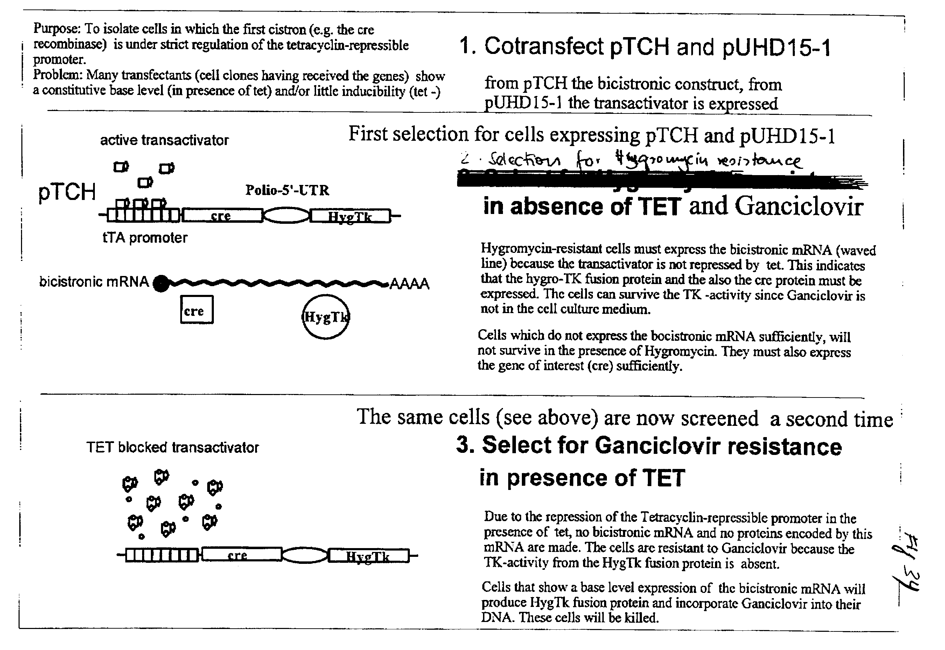

cAg/sAg (Fig. 10),
L1/sAg (Fig. 16),
cAg/mu-IL2 (Fig. 26),
cAg/mu-IL4 (Fig. 27),
cAg/mu-IL7 (Fig. 28),
cAg/mu-IL15 (Fig. 29),
cAg/mu-GM-CSF (Fig. 32),
cAg/mu-IFNg (Fig. 33),
sAg/mu-IL2,
sAg/mu-IL4,
sAg/mu-IL7,
sAg/mu-IL15,
sAg/mu-GM-CSF,
sAg/mu-IFNg,
SV40cT-Ag/mu-L2,
SV40cT-Ag/mu-L4,
SV40cT-Ag/mu-IL7,
SV40cT-Ag/mu-IL15,
SV40cT-Ag/mu-GM-CSF, and

SV40cT-Ag/mu-IFNg.

Another preferred embodiment concerns an expression construct according to the invention, which comprises the following genes:

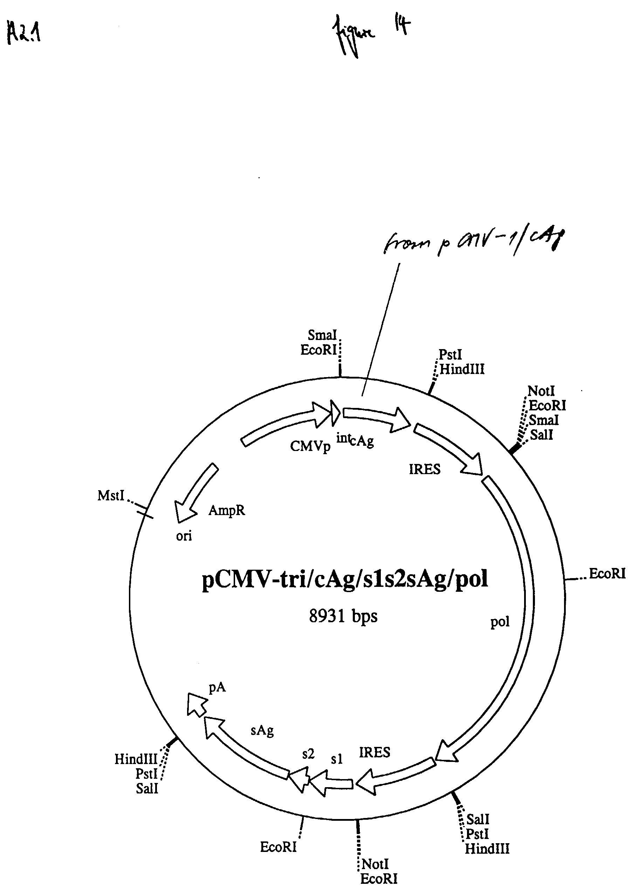

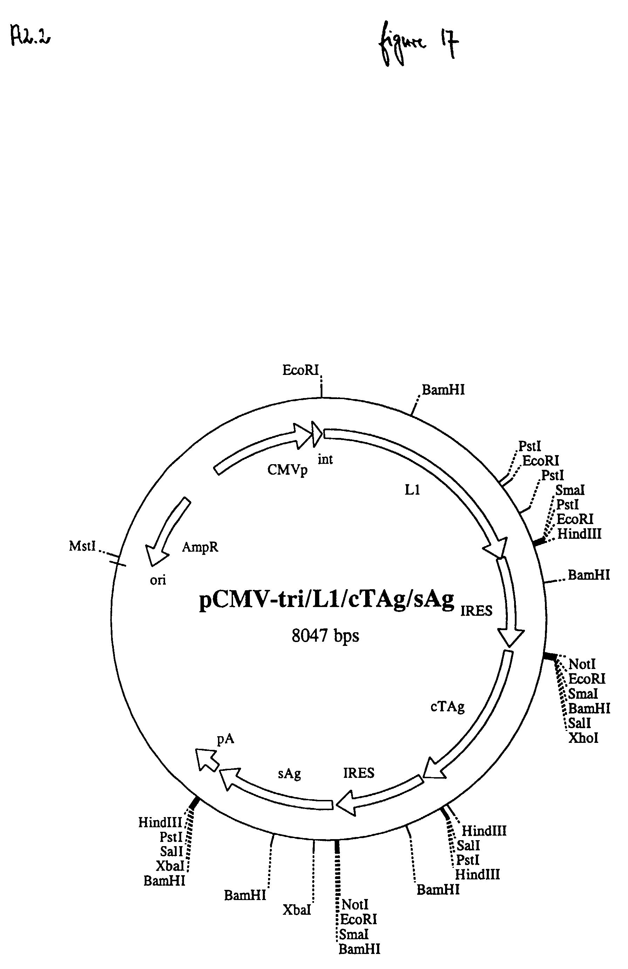

cAg/LS/pol (Fig. 14),
L1/sAg/cTAg (Fig. 17),
cAg/mu-IL12/mu-IL12,
sAg/mu-IL12/mu-IL12, and
SV40cT-Ag/mu-IL12/mu-IL12.

Another preferred embodiment concerns an expression construct according to the invention, which is characterized in that the vector is derived from pSPC-1 or pSPC-2.

Another preferred embodiment concerns an expression construct according to the invention, which is characterized in that the CMVp sequence has been replaced for the SV40p sequence.

Another preferred embodiment concens an expression construct according to the invention, which is characterized by a suicide gene inducible with a therapeutically acceptable drug.

The problem underlaying the invention is further solved by a vaccine which comprises an expression construct according to the invention.

The problem underlaying the invention is further solved by a use of an expression construct according to the invention for the production of a vaccine.

Coexpression of different proteins in stochiometrically defined ratios within a single cell can be achieved by polycistronic expression constructs. Following the intramuscular inoculation of 'naked' plasmid DNA encoding an antigen, humoral and cellular immune responses against the respective antigen expressed by the construct can be primed. The use of polycistronic nucleotide vectors in DNA-based immunization allows the use of three novel options for genetic immunizations.

i. Polycistronic nucleotide vectors can be used to deliver with a single injection a multivalent vaccine that efficiently stimulates a broad spectrum of immune reactivities against two or more different antigens from the same or different pathogens.

ii. Polycistronic nucleotide vectors can be used to code-liver an antigen and a cytokine. Coexpression of the antigen and a cytokine by the same presenting cell provides an 'adjuvants effect' that enhances the magnitude of different immune reactivities and modulates the type of cellular immune effector functions specifically stimulated by the vaccine.

iii. Polycistronic vectors can be constructed that limit the life span of the *in vivo* transfected cell. This is achieved by coexpressing an inducible suicide gene within the antigen-presenting cell. The construct thereby allows expression of the antigen for a few weeks (sufficient to prime an immune response) but allows subsequent elimination of cells expressing the 'foreign' expression constructs.

iv. Polycistronic expression constructs can be used in genetic immunizations that combine the properties listed under i-iii.

By using polycistronic expression plasmids for DNA immunization, the present invention offers the possibility to design recombinant subunit vaccines that:

i. efficiently elicit cytotoxic (ie killer) and helper T-lymphocyte responses as well as antibody responses against two or more different antigens of the same or different pathogens by a single injection;

ii. enhance the immune response and selectively modulate the specific stimulation of selected immune effector functions by the coadministration of cytokines;

iii. enhance the safety of DNA-based vaccination by introducing an inducible cytocidal mechanism into the expression construct that allows elimination of the *in vivo* transfected cells after priming of the immune response.

## Summary of the invention

**1.** *Polycistronic expression constructs as DNA-based, polyvalent vaccines.* Two or more different antigens can be coexpressed from a single transcript in the same cell using polycistronic expression constructs. DNA-based immunization with these constructs stimulates humoral and cellular immune responses against all expressed antigens.

**A.** This approach makes it possible to encode in a single expression construct most immunogenic proteins derived from small viruses (*e.g.*, pathogenic strains of hepadna, papova, or retro virus). The approach avoids the well known risks involved in the use of replicating attenuated or recombinant virus strains for vaccination. DNA-based antigen delivery allows priming of antibody responses and CD4$^+$ T cell responses by secreted, exogenous antigens, and priming of CD8$^+$ T cell responses by intracellular, endogenous antigens.

**B.** The approach allows the construction of polyvalent vaccines that induce immunity to different pathogens by a single DNA injection. Immunogenic protein antigens from different pathogens can be cloned into the same polycistronic expression construct. Furthermore, different fusion constructs encoding chimeric proteins composed of immunogenic domains of different virus proteins can be coexpressed with this technique.

Coexpression of different antigens in the same antigen-presenting cell facilitates priming of clonally diverse T cell populations. Interactions between CD4$^+$ 'helper' T cells and CD8$^+$ 'killer' T cells with the same, specialized presenting cell are known to enhance priming and clonal expansion of specifically stimulated CD8$^+$ T cell populations. Immunization with polycistronic expression constructs has therefore an 'adjuvants effect' that augments MHC class I-restricted CD8$^+$ T cell responses.

**2.** *Enhancement and modulation of cellular immune responses by coexpressing antigen and cytokine in a single presenting cell.* The use of polycistronic constructs in DNA immunizations allows the simultaneous expression of the antigen and a cytokine in the same presenting cell. When DNA encoding both genes is injected on separate plasmids, coexpression of antigen and cytokine in the same cell at the same time is a rare event. The use of polycistronic constructs is a much more efficient technique to target the cytokine activity to the presenting cell during T cell priming.

The cytokine milieu that prevails during T cell priming has a decisive influence on the functional phenotype of the ensuing T cell effector functions. IL-2, IL-12 and IFN-$\tau$ favor TH1-type CD4$^+$ and CD8$^+$ T cell expansion and suppress TH2-type CD4$^+$ T cell maturation. In contrast, IL-4, IL-6 and IL-10 suppress TH1-type CD4$^+$ and CD8$^+$ T cell expansion and facilitate TH2-type CD4$^+$ T cell maturation. Cytokines that attract T cells (IL-15), enhance presenting functions of macrophages and dendritic cells (GM-CSF, IFN-$\tau$), enhance processing and MHC molecule expression (IFN-$\tau$), facilitate early activation of T cells (IL-7) enhance T cell responses. Alternative types of T cell immunity can, either protect the organism against a pathogen, or can mediate an immunopathology associated with the respective infection. With a given pathogen, it is hence important to prime the correct type of T cell response.

Coexpression of antigen and cytokine by the same presenting cell after immunization with polycistronic constructs therefore allows (i) the enhancement of the immune response, and (ii) the selective enhancement or suppression of particular T cell-mediated effector functions (*e.g.*, cytotoxicity, profile of release cytokines, isotype profil of antibody response) specifically activated during the response.

**3.** *Polycistronic vectors allow inducible elimination of transfected presenting cells.* Long-term persistence of *in vivo* transfected cells is not necessary to prime an immune response, and may represent a health hasard. It is therefore desirable to eliminate all cells carrying 'foreign' DNA after the establishment of the immune response. In addition to the antigen, an inducible 'suicide' gene can be coexpressed in the polycistronic construct. Induction of the 'suicide' gene with a drug can selectively eliminate *in vivo* the transfected cell that expresses the genes of the polycistronic construct.

**4.** *Construction of polyvalent vaccines with a cytokine adjuvants that can be eliminated from the organism after inducing an immune response.* Construction of a polycistronic expression construct that combines expression of antigens, a cytokine, and an inducible suicide gene will allow to combine all three properties listed under i-iii above.

**Detailed description of the invention**

**A.** Construction of polycistronic expression vectors

**A1** pCMV promoter insertion and construction of polycistronic expression vectors

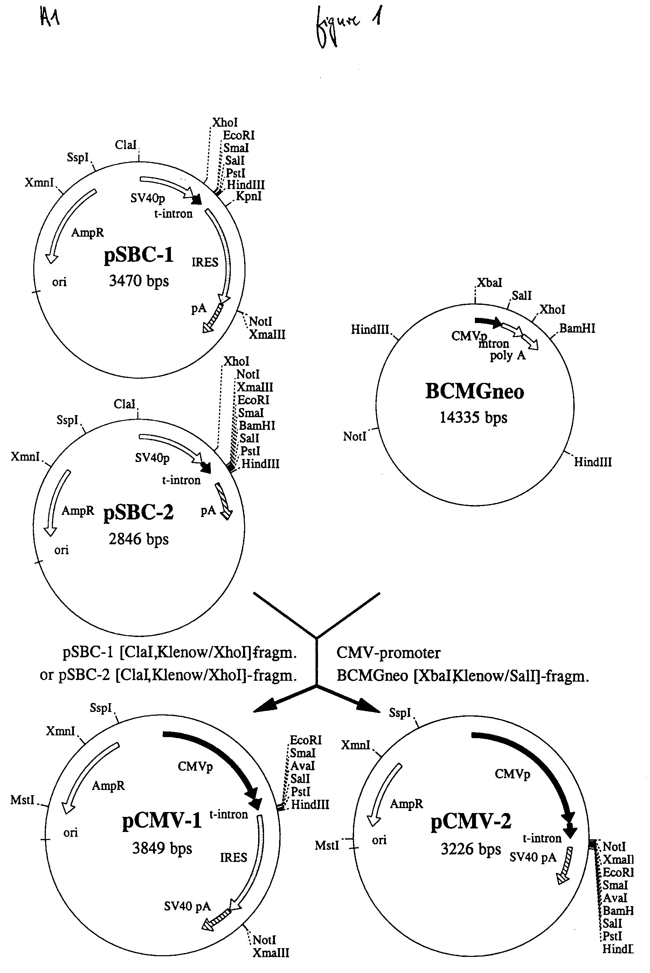

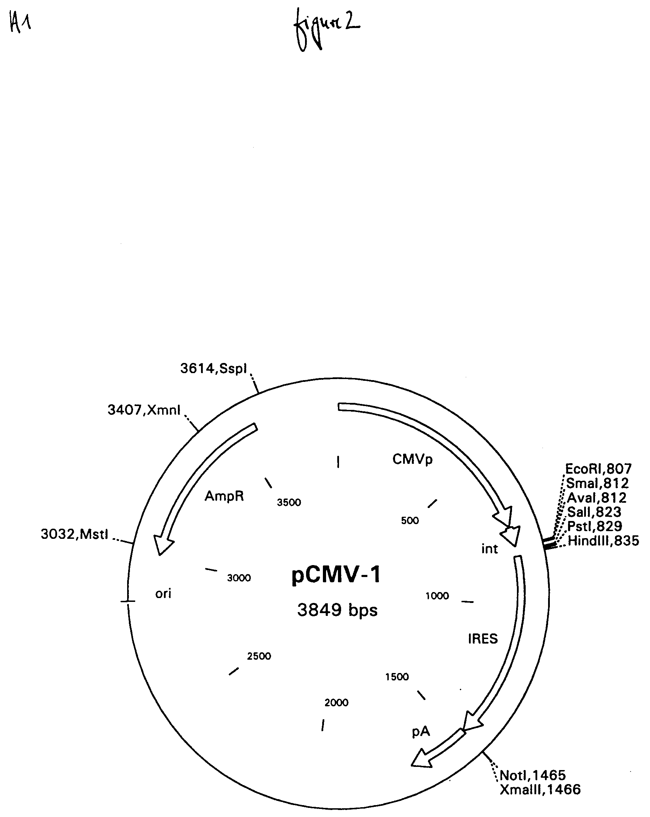

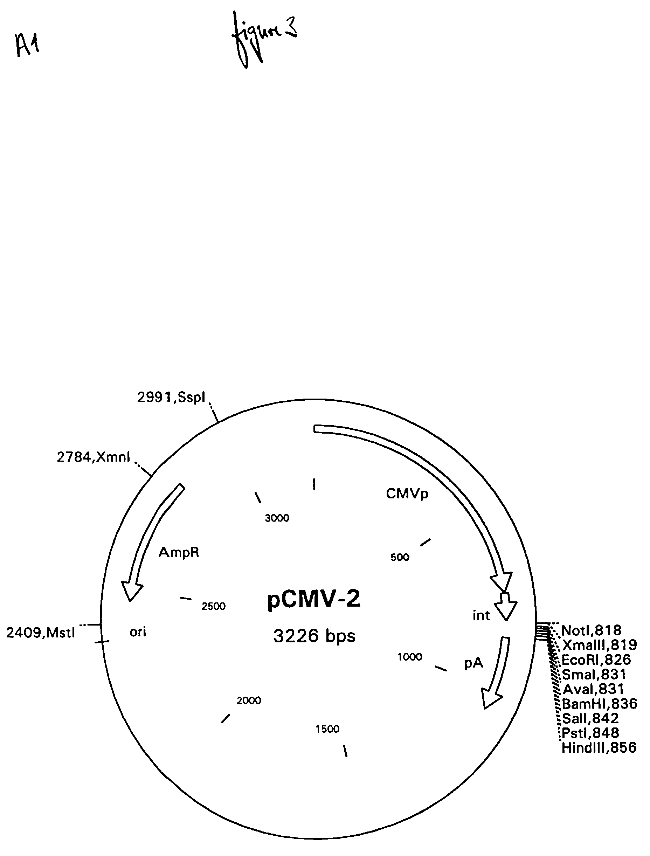

-    Promoter sequences of the immediate early region of the human cytomegalovirus (CMV) have been shown to support expression of an immunogenic gene product after intramuscular injection of plasmid DNA. SV40p sequences in the expression plasmids pSBC-1 and pSBC-2 were replaced by CMVp sequences. This generated the plasmids pCMV-1 and pCMV-2 **(Fig.1)**.
-    The maps of pCMV-1 and pCMV-2 **(Fig.2 and 3; Legend 2 and 3)**
-    The vector fusion between pCMV-1 and pCMV-2 generates the dicistronic pCMV-di as described in **WO94/05785**

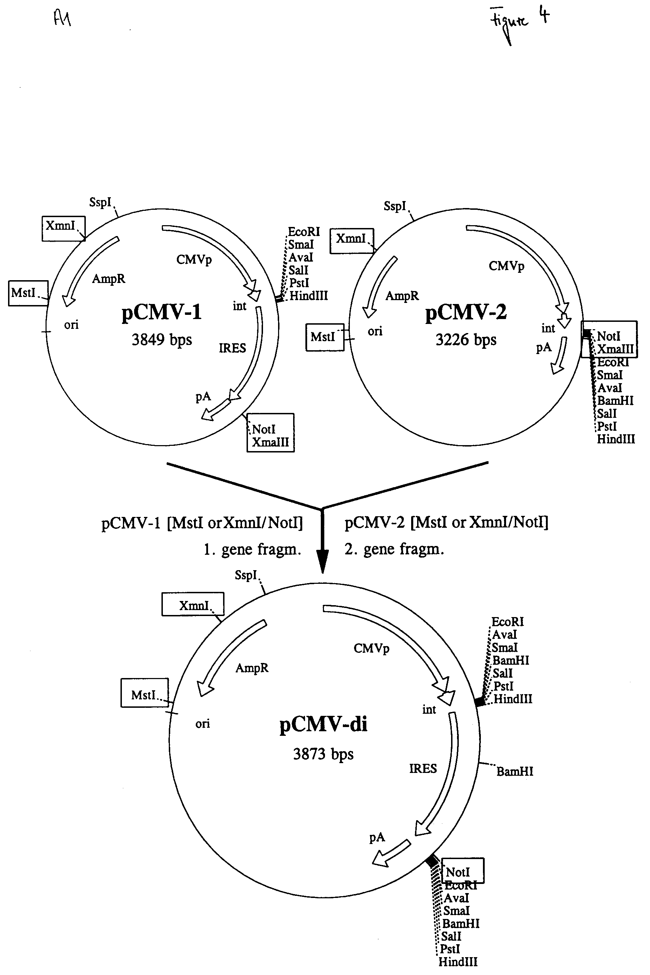

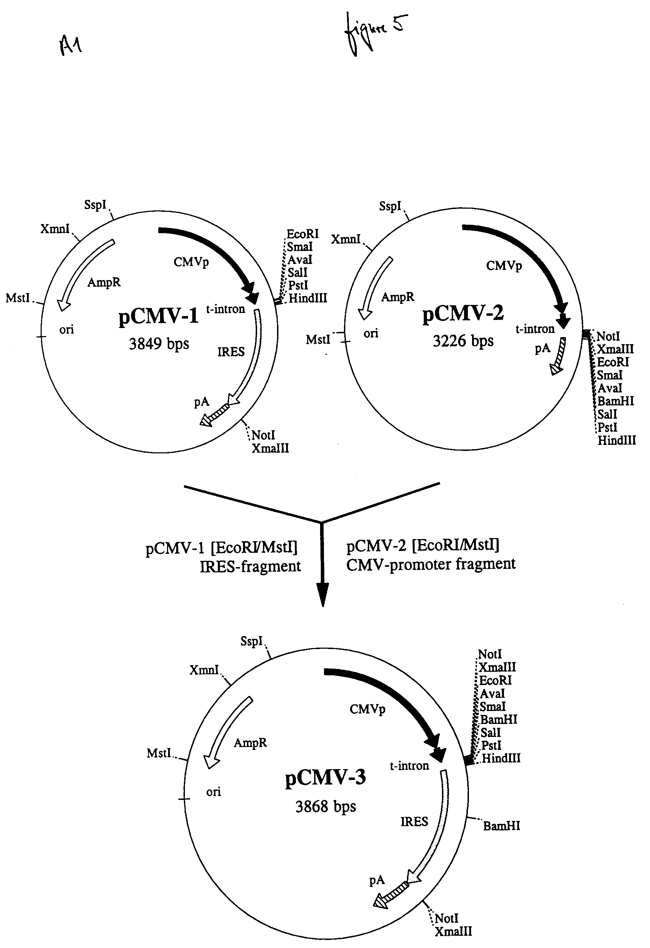

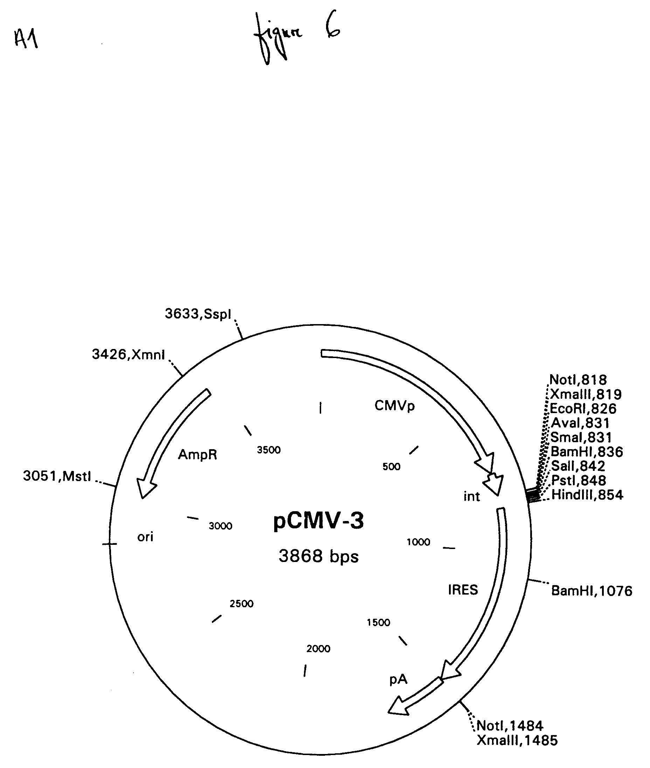

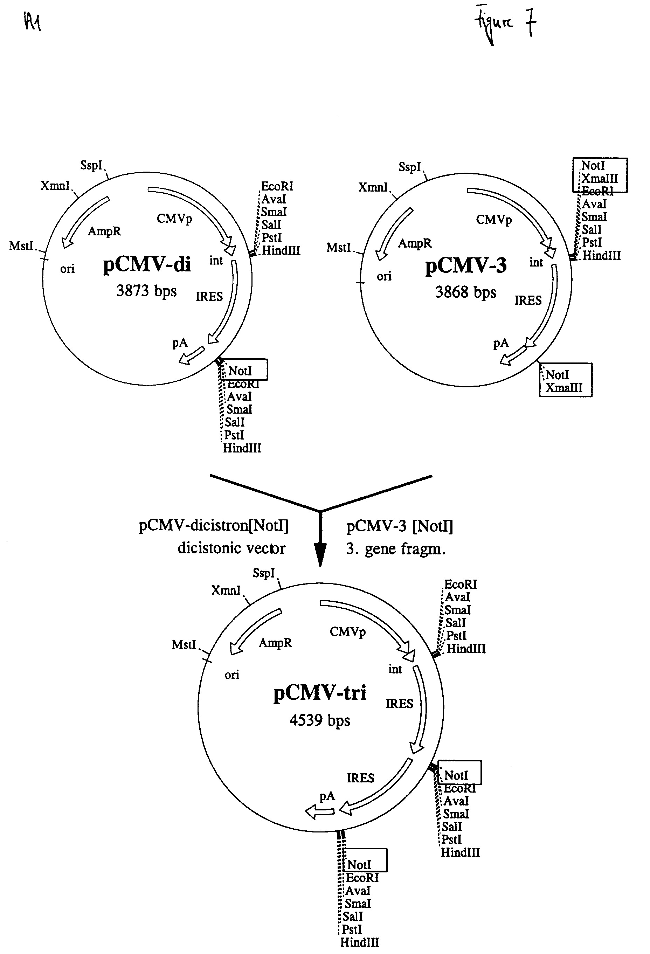

**(Fig.4; Legend 4)**
- An alternative fusion between pCMV-1 and pCMV-2 generates the vector pCMV-3 **(Fig.5; Legend 5)**
- The map of pCMV-3 **(Fig.6)**
- The fusion between pCMV-di and pCMV-3 generates the tricistronic vector pCMV-tri **(Fig.7; Legend 7)**.

**A2** Polycistronic expression constructs as polyvalent vaccines

**A2.1** Polycistronic expression constructs that express most proteins from a small virus

*Examples*:

**Hepatitis B Virus (HBV)**

    *core* **antigen (nucleocapsid, c-Ag or HBcAg)**
    *small surface* **protein (sAg) and large surface antigen (S1S2sAg or LS)**
    *polymerase* **(pol)**

**Human papilloma virus (HPV)-16** *surface* **protein L1**
**Simian (papova) virus (mutant cytoplasmic) large tumor antigen (cT or cT-Ag)**

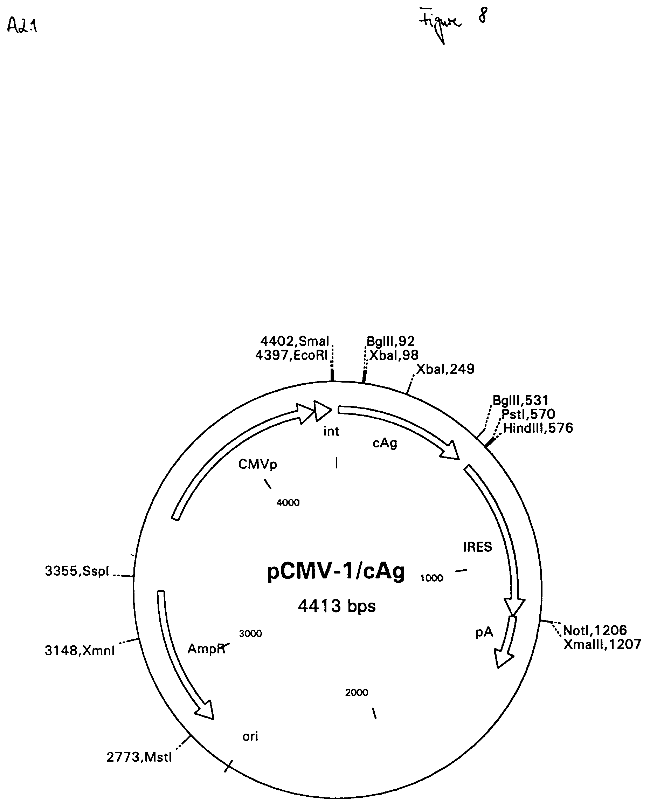

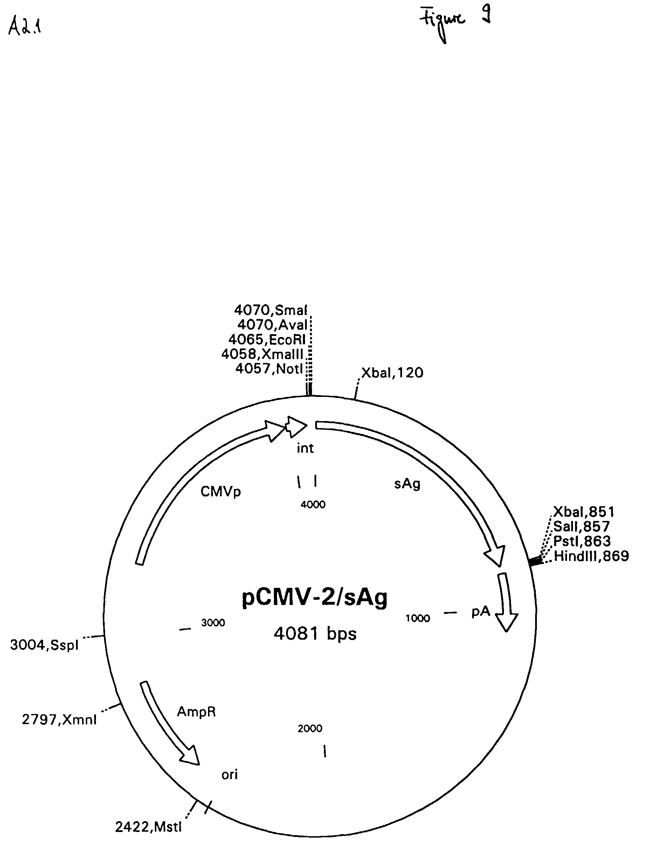

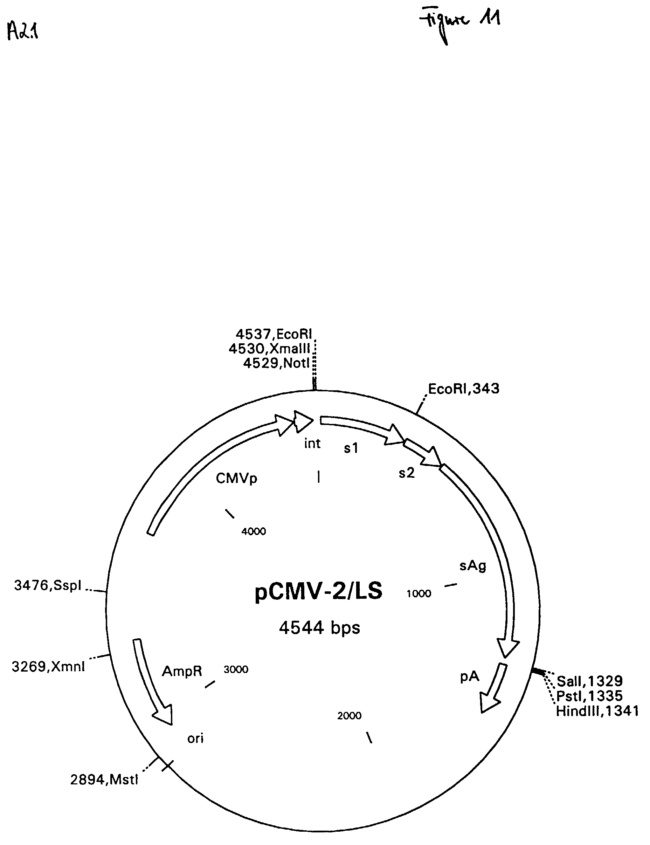

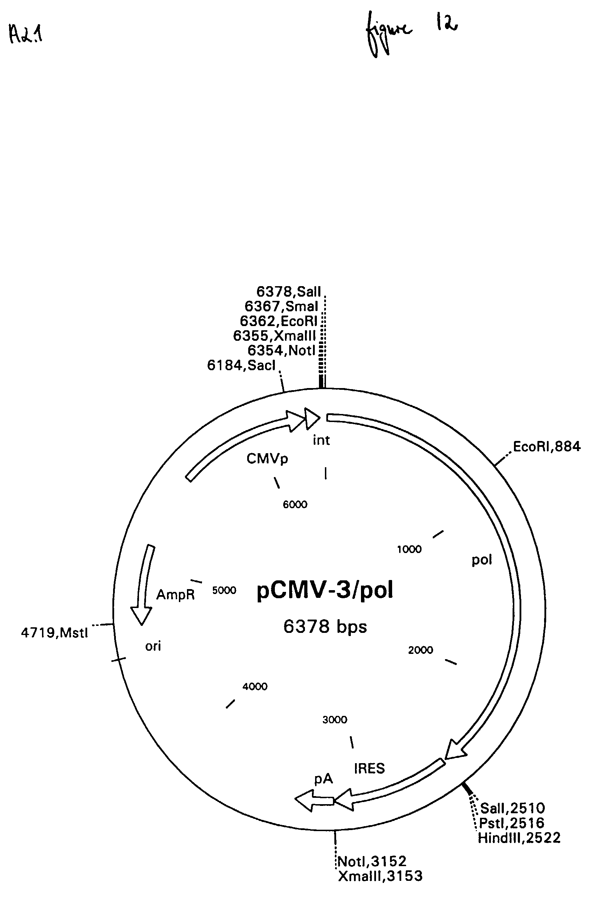

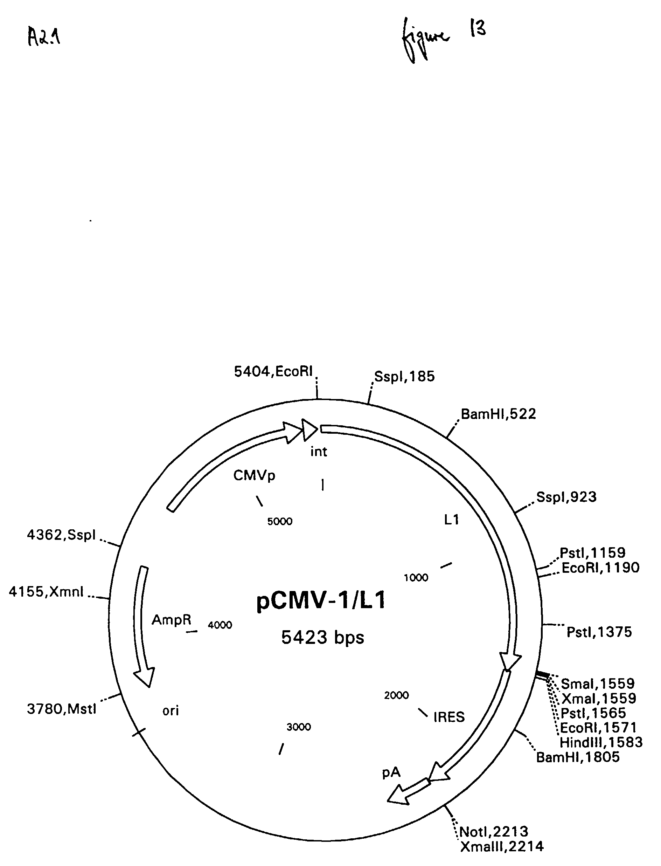

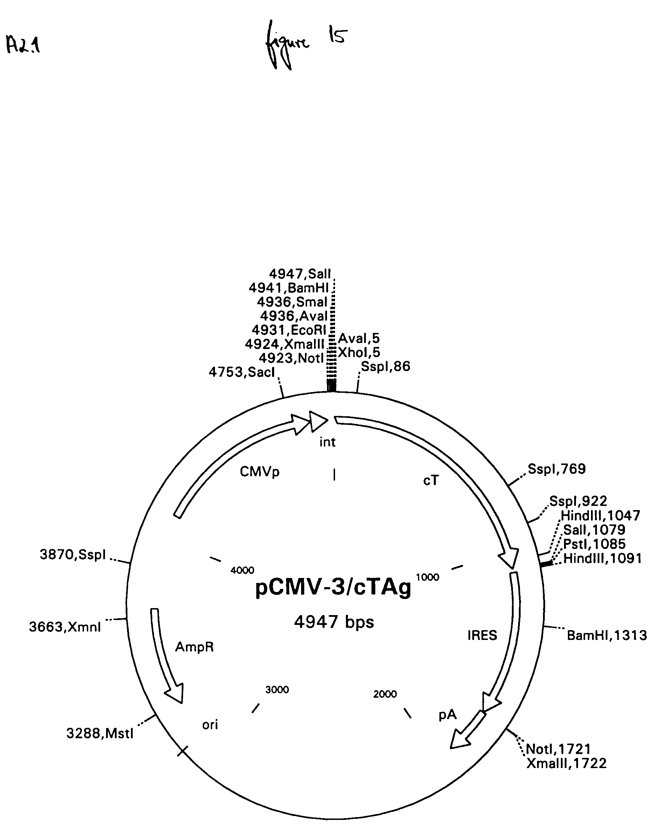

- Cloning of the HBV core antigen (cAg) into pCMV-1 **(Fig.8; Legend 8)**
- Cloning of the HBV small surface antigen (sAg) into pCMV-2 **(Fig.9; Legend 9)**
- construction of the dicistronic vector pCMV-di/cAg/sAg containing the HBV core antigen (cAg) in the 1. cassette, and the HBV small surface antigen (sAg) in the 2. Cassette **(Fig.10)**
- Cloning of the HBV large surface antigen (LS) into pCMV-2 **(Fig.11; Legend 11)**
- Cloning of the HBV polymerase (pol) into pCMV-3 **(Fig.12; Legend 12)**\*
- Cloning of the HPV surface antigen (L1) into pCMV-1 **(Fig.13; Legend 13)**; also fig. 16
- construction of the tricistronic vector pCMV-tri/cAg/LS/pol containing the HBV core antigen (cAg) in the 1. cassette, and the HBV large surface antigen (LS) in the 2. cassette, and the HBV polymerase (pol) in the 3. cassette **(Fig.14)**\*
- Cloning of the SV40 cT-Ag into pCMV-3 **(Fig.15; Legend 15)**\*

**A2.2** Polycistronic expression constructs as polyvalent vaccines that encode protein antigens from different pathogens

- Construction of bicistronic plasmid **pCMV-di/L1/sAg (Fig.16)**\*
- Construction of tricistronic plasmid **pCMV-tri/L1/sAg/cTAg (Fig.17)**\*

**A3** Coexpression of antigen and cytokine from a polycistronic construct

**A3.1** Expression of cytokines in pCMV-2

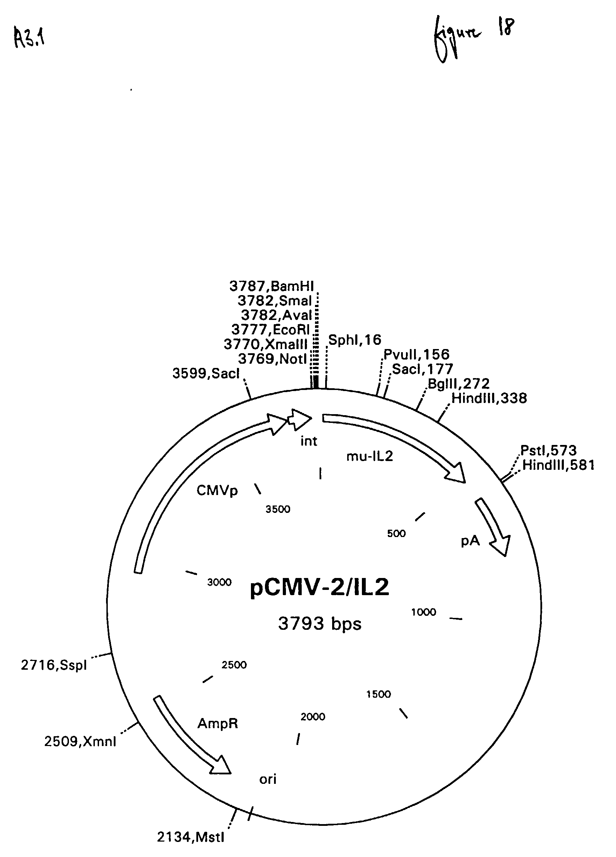

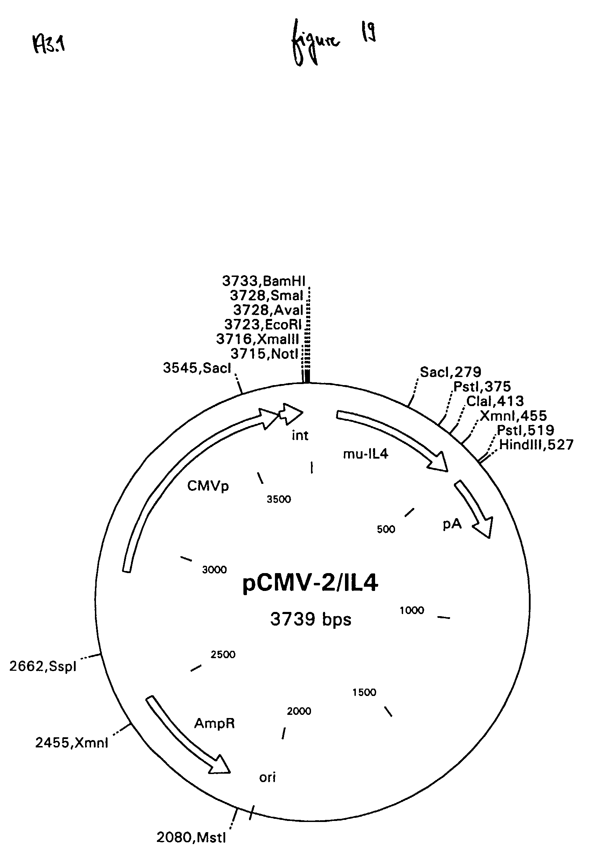

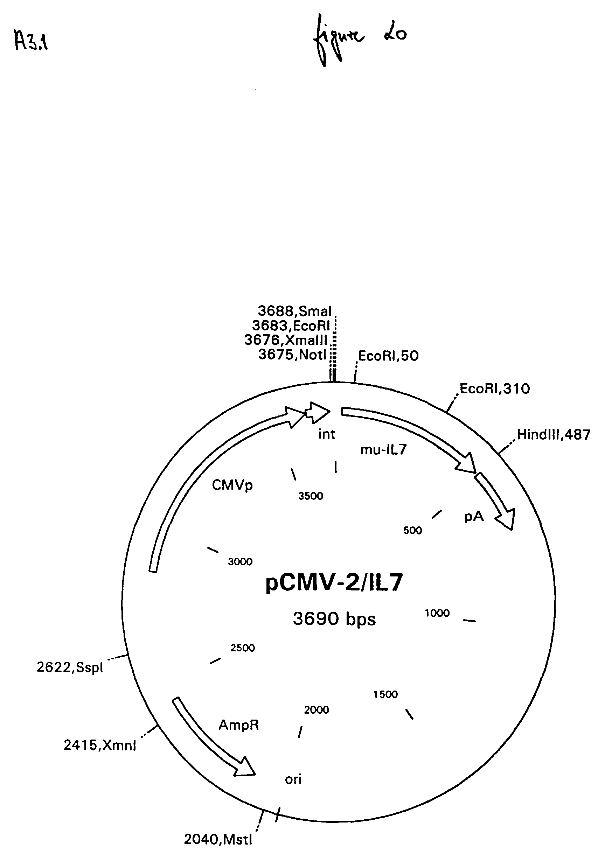

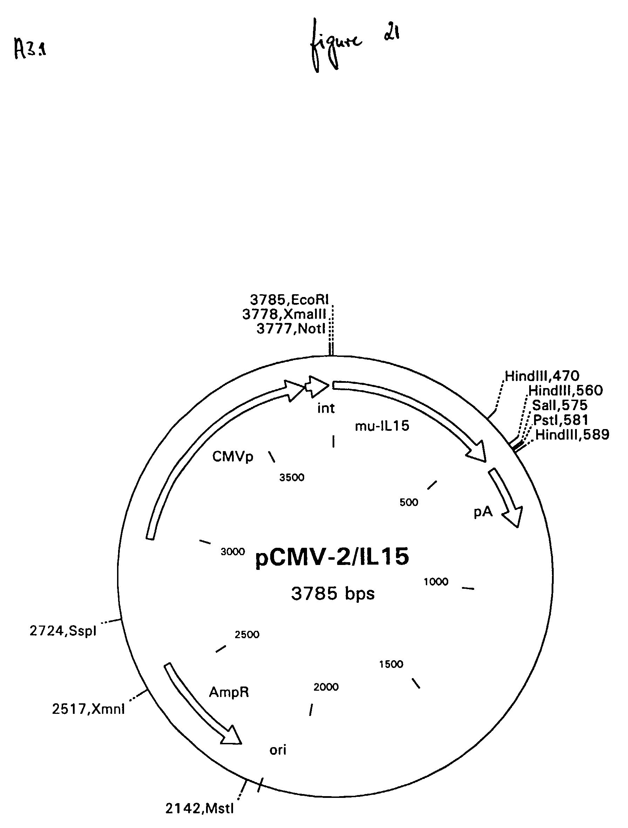

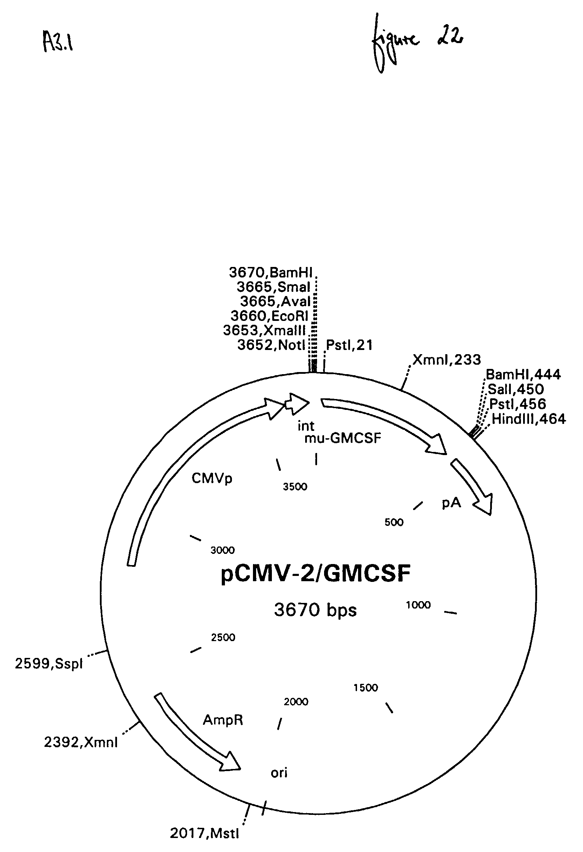

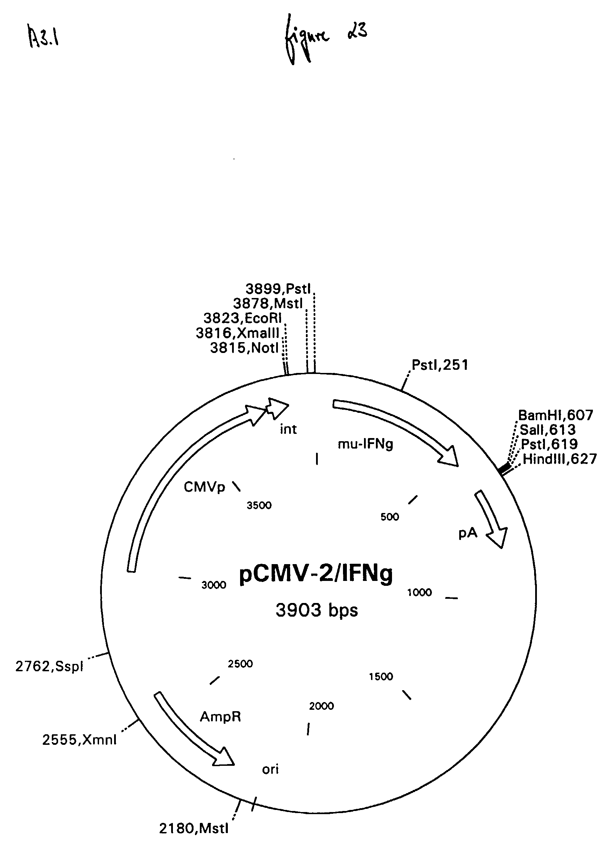

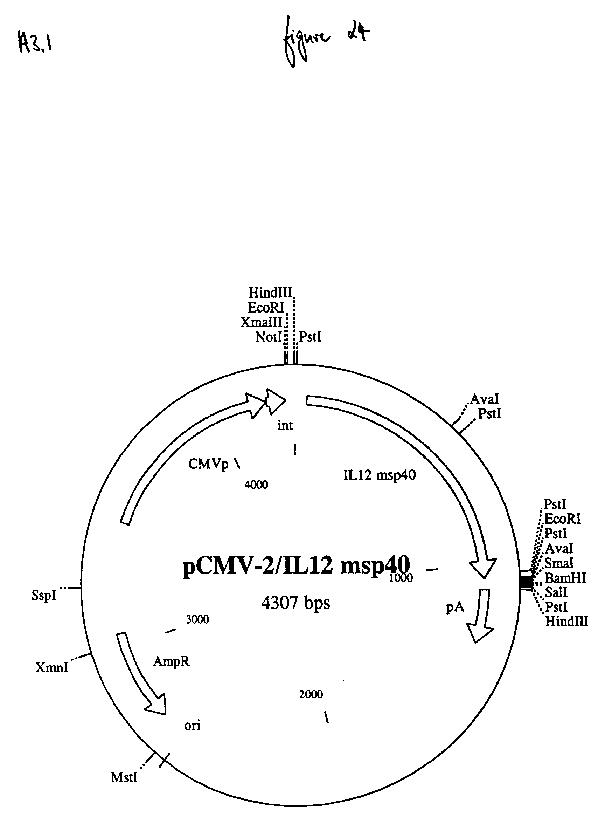

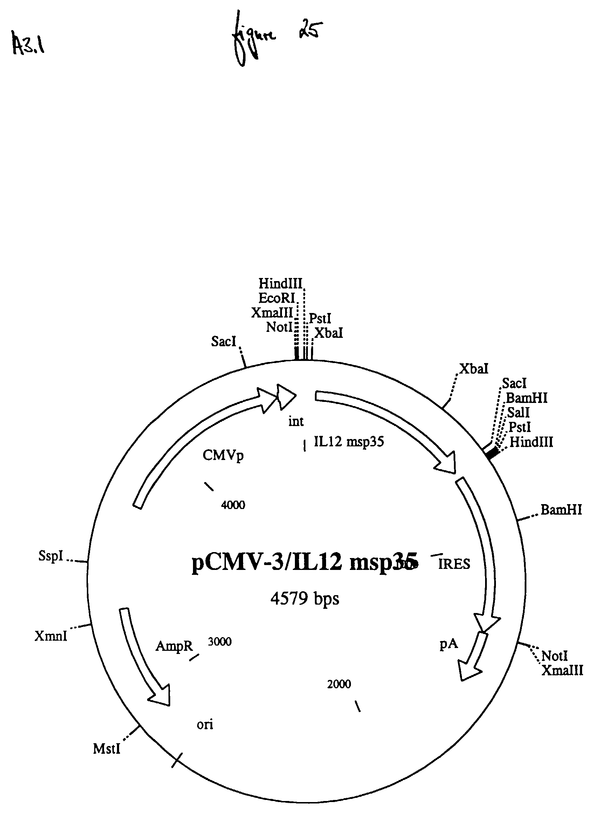

- expression of the murine interleukin-2 (**mu-IL2**) gene in pCMV-2 **(Fig.18; Legend 18)**
- expression of the murine interleukin-4 (**mu-IL4**) gene in pCMV-2 **(Fig.19; Legend 19)**
- expression of the murine interleukin-7 (**mu-IL7**) gene in pCMV-2 **(Fig.20; Legend 20)**
- expression of the murine interleukin-15 (**mu-IL15**) gene in pCMV-2 **(Fig.21; Legend 21)**
- expression of the murine granulocyte-macrophage colony stimulating factor (**mu-GM-CSF**) gene in pCMV-2 **(Fig.22; Legend 22)**
- expression of the murine interferon-g (**mu-IFNg**) gene in pCMV-2 **(Fig.23; Legend 23)**
- expression of the murine interleukin-12 (**mu-IL12**) gene: IL12msp40 in pCMV-2 **(Fig.24; Legend 24)**; IL12msp35 in pCMV-3 **(Fig.25; Legend 25)**.

**A3.2** Construction of dicistronic and tricistronic plasmids coexpressing an a viral antigen and a cytokine

Construction of di- or tricistronic expression plasmids containing the HBV **cAg** in the 1. Cassette and

- the murine interleukin-2 (**mu-IL2**) in the 2. Cassette **(Fig.26; Legend 26)**
- the murine interleukin-4 (**mu-IL4**) in the 2. Cassette **(Fig.27; Legend 27)**
- the murine interleukin-7 (**mu-IL7**) in the 2. Cassette **(Fig.28; Legend 28)**
- the murine interleukin-15 (**mu-IL15**) in the 2. Cassette **(Fig.29; Legend 29)**

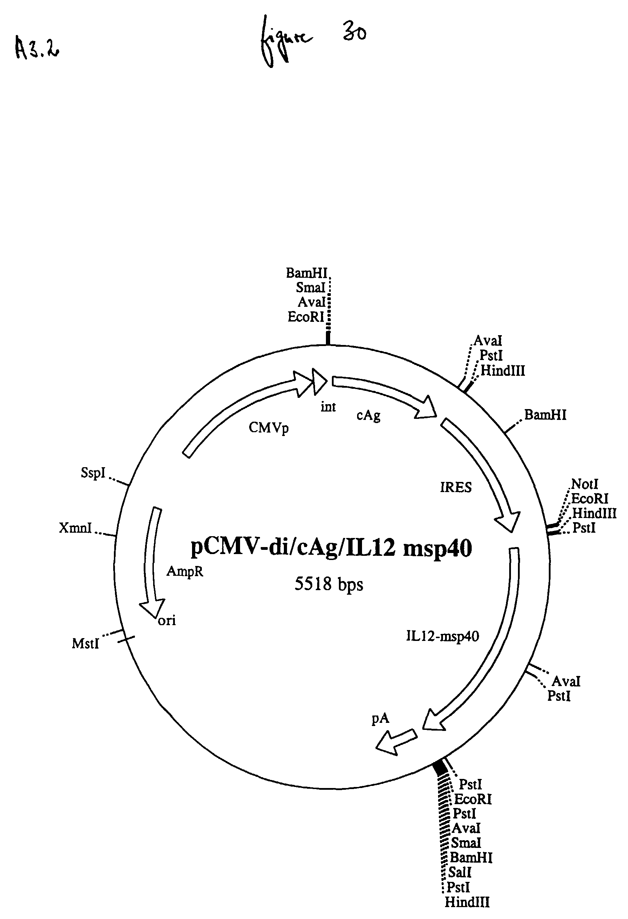

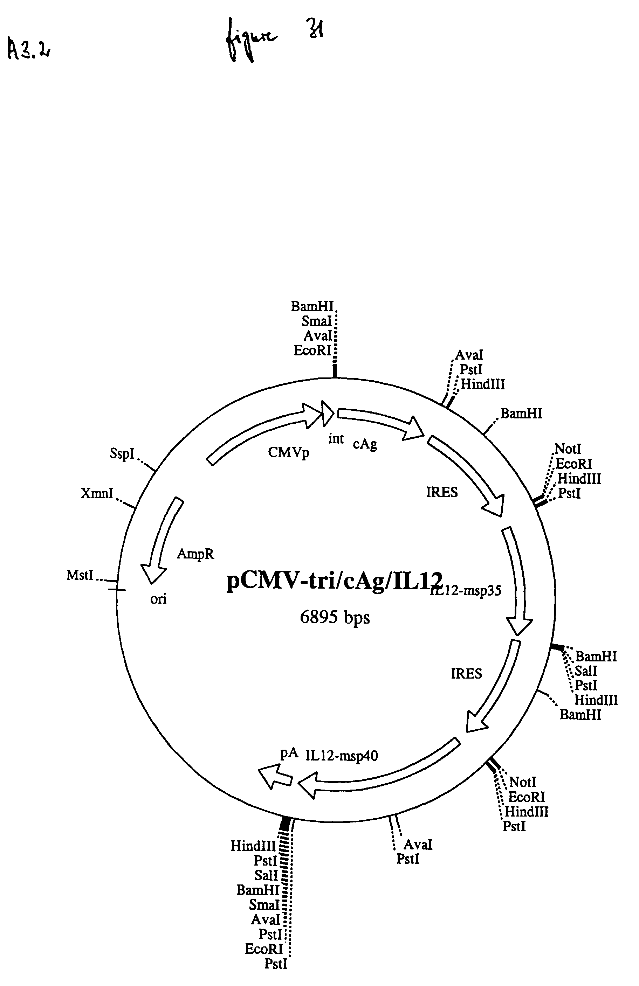

- the murine interleukin-12 (**mu-IL12**) in the 2. and 3.Cassette (**Fig.30 and 31; Legend 30 and 31**)
- the murine granulocyte-macrophage colony stimulating factor (**mu-GM-CSF**) in the 2. Cassette (**Fig.32; Legend 32**)
- the murine interferon-g (**mu-IFNg**) in the 2. Cassette (**Fig.33; Legend 33**)

Similar constructs were generated containing the HBV small surface antigen (sAg) or the SV40 cT-Ag in the 1. cassette.

**A4** Example of Expression by Means of Tricistronic Expression Vectors in Mammalian Cells

A tricistronic expression vector was constructed for the expression of recombinant antibody in mammalian cells. The vector contains a strong MPSV (Myoproliferative Sarkoma Virus) Promotor which drives an expression unit containing the following elements:

The first cistron consists of IgG light chain antigen followed by an IRES element (Poliovirus derived), followed by an IgG heavy chain, followed by a second (identical) IRES element, followed by a puromycin resistance gene (puromycin acetyl transferase), followed by a polyAdenylation signal for termination of primary transcripts. This expression unit is integrated in a simple pBR-based vector backbone.

This construct was introduced into BHK-21 cells. Puromycin resistant cell clones were isolated and IgG expression up to 5 μg/ml.24 hours (heterodimeric) could be found. Five cell clones were constantly cultivated in the presence of puromycin. In no case, the expression of IgG was lost. However, when these cell clones were cultivated in the absence of Puromycin, IgG expression declined rapidly. The decrease in IgG expression varied from cell clone to cell clone. A screening of more than 500 cell clones for IgG overexpression and stability revealed a few cell clones which stably expressed IgG in the absence of Puromycin. However, the expression level was below 1 μg/ml.24 hours.

Although the construct contains two identical IRES elements the plasmids seemed to be stable in *E. coli* and mammalian cells.

**A5** Inducible elimination of transfected presenting cells expressing genes encoded by polycistronic vectors

**A5.1** Generation of mammalian cells with protein expression

A number of suicide vectors in bicistronic expression vectors have been constructed and tested in mammalian cells. Basically, all constructs depend on a pBR-based vector backbone with one expression cassette integrated. The cassette contains a promotor (constitutive or regulatable), a gene as a first cistron, followed by an IRES element (Poliovirus derived) and as a second gene (a) the wild-type or (b) fusion protein gene of Herpes Simplex Virus Thymidin kinase gene (HSVtk), followed by a polyAdenylation site.

> **Example a):** The promotor is a constitutive strong MPSV promotor, the first cistron contains a secreted alcaline phosphatase encoding gene (SEAP), the second cistron consists of wild-type HSVtk gene. The vector was co-transfected together with a selectable marker (Puromycin resistance gene) into mammalian BHK-cells and Puromycin resistant cell clones were isolated. Most cell clones expressed SEAP to a different extent. When these cells were treated with Ganciclovir, most of the cells died and in all cases, SEAP expression was not detectable anymore.

> **Example b):** The expression construct is identical to a) with the exception that instead of wt (wild type) HSVtk a fusion protein containing a hygromycin resistance gene fused to the HSVtk gene was used. In this case, the cells were selected for Hygromycin resistance after transfection with this singular construct. In all cell clones, SEAP expression was detected. Ganciclovir selection eliminated all cells.

**A5.2** Generation of mammalian cells with conditional expression of *cre* recombinase

We have developed a simple strategy of generating cells with an adjustable cre expression controlled by a Tetracyclin (TET) responsive promotor (5). This promotor is regulated by a co-expressed transactivator which is inactivated by TET. The successful application of this conditional expression system requires the screening for single cell clones in which the transferred gene is strictly regulated. According to our experience the frequency of generating such cell clones is below 1 in 100, which reflects the number of suitable integration sites within the host cell chromosomal DNA. To simplify the protocol for screening for cells with strict TET regulated expression we combined the TET-responsive expression system with a positive/negative selection procedure (**figure 1**):

In pTCH, a derivative of pTBC (7), the tTA promotor controls expression of a bicistronic transcription unit (8). *Cre* represents the first cistron, the second cistron encodes hygtk, a fusion protein of Hygromycin B phosphotransferase and thymidine kinase (tk) (a gift of C. Karreman). *Cre* expressing cells are generated by co-transfection of NIH3T3 cells with pTCH and pUHD15-1 encoding the TET-respressible transactivator TA (5). By selection for Hygromycin B resistant cells in the absence of TET, transfectants are isolated in which both, TA and *cre*, are stably integrated and expressed. To eliminate cell clones in which cre is expressed in the presence of TET - which is frequently observed as a consequence of integration into unfavourable sites of host DNA, Hygromycin B resistant cells were cultivated in presence of both TET and Ganciclovir but in the absence of Hygromycin B. Using this procedure, cells which still transcribe the bicistronic expression unit are eliminated by counterselection against tk activity with Ganciclovir. To further circumvent the survival of cells in which tk expression is repressed by methylation, cells may be additionally exposed to 5-Azacytidine. This was shown to be successful in two of the experiments (data not shown).

Hygromycin B selected HIH3T3 cells show absence of *cre* expression in the presence of TET (Ganciclovir resistant cells). Using this procedure cell populations can be generated that allow a precise and tight conditional control of *cre* expression. In **table 1** we show the power of this system in 5 mixtures of *cre*-expressing transfectants, which were infected with a retroviral vector carrying a *loxP-neo-loxP* cassette in absence and presence of TET. The excision ratio reflects the level of *cre* activity. If compared to a single positive selection the positive/negative selection procedure enhances excision ratio more than 100-fold (data not shown). The time course of *cre*-mediated excision efficiency on a chromosomal target was determined (**table 2**).

**TABLE 1.**

**Regulation of cre activity by TET**

Clone mixtures (M1-M5) of pTCH/pUHD15-1 co-transfected NIH3T3 cells generated according to the protocol (c.f. **figure 1**) were infected with pBPLNL in presence or absence ot TET. pBPLNL was derived from pBabePuro (6) by integration of a *loxP-neo-loxP* cassette. In the absence of cre the vector confers resistance to G418. Relative cre activity is given by the ratio of clone numbers obtained after selection of infected cells with G418+TET (absence of *cre*) and G418+Hygromycin B (presence of *cre*).

TABLE 1

| clone mixtures | M1 | M2 | M3 | M4 | M5 |
|---|---|---|---|---|---|
| relative *cre* activity | ≥700 | > 70 | ≥800 | ≥100 | ≥100 |

**TABLE 2**

**Time course of cre activity on proviral targets.**

pTCH/pUHD15-1 co-transfected cells were selected according to the protocol outlined in **figure 1**. The cells were infected with pBPLNL and selected for integration of complete proviruses by growth in presence of G418 and TET (absence of *cre*). On day 0, resistant cells were directly seeded in medium supplemented with G418+Hygromycin B (presence of *cre*) or G418+TET (absence of *cre*). Specific excision is defined by the percental ratio of cell clones obtained after selection in these media. To allow *cre* expression preliminary to selection, G418 resistant cells were cultivated in absence of TET and G418 for indicated times (2, 7 and 31 days) and subsequently seeded in the selection media (G418+Hygromycin B and G418+TET).

TABLE 2

| times (days) of *cre* expression | 0 | 2 | 7 | 31 |
|---|---|---|---|---|
| % excision | 33 | 90 | 97 | 99 |

**REFERENCES**

1 Sauer, B. (1994) *Curr. Opin. Biotechnol. 5*, 521-527

2 Smith, A. J. H., De Sousa, M. A., Kwabi-Addo, B., Heppell-Parton, A., Impey, H. and Rabbitts, P. (1995) *Nature Genet.* 9, 376-385

3 Baubonis, W. and Sauer, B. (1993) *Nucleic Acids Res. 21*, 2025-2029.

4 Metzger, D., Clifford, J., Chiba, H. and Chambon, P. (1995) *Proc. Natl. Acad. Sci. USA 92*, 6991-6995.

5 Gossen, M., and Bujard, H. (1992) *Proc. Natl. Acad. Sci USA 89*, 5547-5551

6 Morgenstern, J. P. and Land, H. (1990) *Nucleic Acids Res. 18*, 3587-3596.

7 Kirchhoff, S., Köster, M., Wirth, M., Schaper, F., Gossen, M., Bujard, H. and Hauser, H. (1995) *Trends Genet. 11*, 219-220.

8 Dirks, W., Wirth, M. and Hauser, H. (1993) *Gene 12*, 247-249.

**B.** Use of the vectors in vivo in genetic immunization experiments

**B1** Humoral and cellular immune response to viral antigens induced by genetic immunization

| # | mice immunized with plasmid | μg DNA/mouse | cytotoxic T response[1] | | humoral response[2] |
|---|---|---|---|---|---|
| | | | Ag$^+$ target | Ag$^-$ target | |
| 1 | pCMV-1/cAg | 100 | $43 \pm 11$ | $4 \pm 3$ | $1.8 \pm 0.5$ |
| 2 | pCMV-1/cAg | 10 | $12 \pm 8$ | $8 \pm 5$ | <0.1 |
| 3 | pCMV-1/cAg | 1 | $7 \pm 5$ | $5 \pm 4$ | <0.1 |
| 4 | pCMV-2/sAg | 100 | $89 \pm 5$ | $4 \pm 3$ | $28.000 \pm 1650$ |
| 5 | pCMV-2/sAg | 10 | $22 \pm 11$ | $3 \pm 2$ | $935 \pm 560$ |
| 6 | pCMV-2/sAg | 1 | $6 \pm 5$ | $5 \pm 4$ | <50 |

Mice (5 animals per group) were injected intramuscularly with 'naked' plasmid DNA. In 1-3, C57BL/6 (H-2$^b$) mice were immunized. In 4-5, BALB/c mice (H-2$^d$) were immunized. The response was read out 4 weeks post-immunization. Mean values ($\pm$ SD) are given.

[1] *Cytotoxicity assay for specific T cell reactivity*. Spleen cells from immunized mice were suspended in a-MEM tissue culture medium supplemented with 10 mM HEPES buffer, $5 \times 10^{-5}$ M 2-ME, antibiotics and 10% v/v fetal calf serum. $3 \times 10^7$ responder cells were cocultured with $1.5 \times 10^6$ syngeneic, HBcAg-expressing RBL5/C or HBsAg-expressing P815/S transfectants (irradiated with 20.000 rad) in 10 ml medium in upright 25 cm$^2$ tissue culture flasks in a humidified atmosphere/7% CO$_2$ at 37 °C. Cytotoxic effector populations were harvested after varying intervals of *in vitro* culture and washed twice. Serial dilutions of effector cells were cultured with $2 \times 10^3$ $^{51}$Cr-labeled targets in 200 μl round-bottom wells. Specific cytolytic activity of cells was tested in short-term $^{51}$Cr-release assays against transfected (Ag$^+$) or non-transfected (Ag$^-$) control targets. After a 4 h incubation at 37¡C, 100 μl of supernatant were collected for g-radiation counting. The percentage specific release was calculated as [(experimental release - spontaneous release)/(total release - spontaneous release)] x 100 . Total counts were measured by resuspending target cells. Spontaneously released counts were always less than 20 % of the total counts. Data shown represent mean specific lysis values of 5 mice ($\pm$ SD).

[2] *Determination of specific serum antibody levels*. Antibodies against HBcAg were detected in mouse sera using the CORE$^R$ anti-HBc kit (cat.no. 2259-20; Abbott GmbH, Wiesbaden, FRG). Concentrations of anti-HBc were standardized against a reference standard of the Paul-Ehrlich-Institute (Langen, FRG). Anti-HBeAg antibody titers in sera of DNA-immunized mice were determined using the IMX anti-HBe assay (Abbot). This assay was also standardized using a reference from the Paul-Ehrlich-Institute.

Antibodies against HBsAg were detected in mouse sera using the commercially available test IMxAUSAB (Abbott, Wiesbaden, FRG). In this readout, microparticles coated with HBsAg were mixed with mouse serum samples to allow specific binding of anti-HBsAg antibodies. Microparticles coated with antigen-antibody complexes were transferred and irreversibly bound to a glass fibre matrix. Biotinylated recombinant HBsAg was added, and binding to the complex was traced by an alkaline phosphatase conjugated anti-biotin antibody followed by the addition of the substrate 4-methyl-lumbelliferyl-phosphate. Antibody levels were quantified using six standard sera (0 to 1000 mIU/ml). The tested sera were diluted so that the measured OD values were between standard serum one and six. Values presented in this paper are calculated by multiplying the serum dilution with the measured antibody level (mIU/ml). Serum titers shown are the mean of 5 individual mice (± SD).

**B2.1** Polycistronic expression constructs as DNA-based, polyvalent vaccines

Antibody and CTL response to HBsAg and HBcAg after a single DNA immunization with a polycistronic construct

| # | mice immu-nized with plasmid | μg DNA/mouse | cytotoxic T response[1] | | | | humoral response[2] | |
|---|---|---|---|---|---|---|---|---|
| | | | anti-sAg | | anti-cAg | | anti-sAg | anti-cAg |
| | | | P815/S | P815 | RBL5/C | RBL5 | | |
| 1 | pCMV-di/cAg/sAg | 100 | 78 ± 9 | 5 ± 3 | 39 ± 5 | 7 ± 5 | 22.054 ± 2350 | 1.9 ± 6 |
| 2 | pCMV-di/cAg/sAg | 10 | 26 ± 7 | 6 ± 4 | 18 ± 6 | 5 ± 3 | 874 ± 2340 | 0.7 ± 0.4 |
| F1 (BALB/c x C57BL/6) mice were immunized with 'naked' plasmid DNA. Legend as above | | | | | | | | |

[1] *Cytotoxioity assay for speoioif T oell reactivity*. Spleen cells from immunized mice were suspended in a-MEM tissue culture medium supplemented with 10 mM HEPES buffer, $5 \times 10^{-5}$ M 2-ME, antibiotics and 10% v/v fetal calf serum. $3 \times 10^7$ responder cells were cocultured with $1.5 \times 10^6$ syngeneic, HBcAg-expressing RBL5/C or HBsAg-expressing P815/S transfectants (irradiated with 20.000 rad) in 10 ml medium in upright 25 cm$^2$ tissue culture flasks in a humidified atmosphere/7% $CO_2$ at 37 °C. Cytotoxic effector populations were harvested after varying intervals of *in vitro* culture and washed twice. Serial dilutions of effector cells were cultured with $2 \times 10^3$ $^{51}$Cr-labeled targets in 200 μl round-bottom wells. Specific cytolytic activity of cells was tested in short-term $^{51}$Cr-release assays against transfected (Ag$^+$) or non-transfected (Ag$^-$) control targets. After a 4 h incubation at 37¡C, 100 μl of supernatant were collected for g-radiation counting. The percentage specific release was calculated as [(experimental release - spontaneous release)/(total release - spontaneous release)] x 100 . Total counts were measured by resuspending target cells. Spontaneously released counts were always less than 20 % of the total counts. Data shown represent mean specific lysis values of 5 mice (± SD).

[2] *Determination of speoifio serum antibody levels*. Antibodies against HBcAg were detected in mouse sera using the CORE$^R$ anti-HBc kit (cat.no. 2259-20; Abbott GmbH, Wiesbaden, FRG). Concentrations of anti-HBc were standardized against a reference standard of the Paul-Ehrlich-Institute (Langen, FRG). Anti-HBeAg antibody titers in sera of DNA-immunized mice were determined using the IMX anti-HBe assay (Abbot). This assay was also standardized using a reference from the Paul-Ehrlich-Institute.

**B2.2** Polycistronic expression constructs as polyvalent vaccines that encode protein antigens from different pathogens

Antibody and CTL response to HBsAg of HBV and the T-Ag of SV40 after a single DNA immunization with a polycistronic construct

| # | mice immunized with plasmid | μg DNA/mouse | cytotoxic T response[1] | | | | humoral response[2] | |
|---|---|---|---|---|---|---|---|---|
| | | | anti-sAg | | anti-T-Ag | | anti-sAg | anti-T-Ag |
| | | | P815/S | P815 | RBL5/T | RBL5 | | |
| 1 | pCMV-di/sAg/cT-Ag | 100 | $89 \pm 12$ | $5 \pm 3$ | $78 \pm 11$ | $3 \pm 2$ | $7654 \pm 1356$ | n.d. |
| 2 | pCMV-di/sAg/cT-Ag | 10 | $32 \pm 9$ | $8 \pm 5$ | $43 \pm 8$ | $5 \pm 4$ | $665 \pm 212$ | n.d. |

F1 (BALB/c x C57BL/6) mice were immunized with 'naked' plasmid DNA. Legend as above. nd, not detectable (in western)

[1] *Cytotoxicity assay for specioif T cell reactivity*. Spleen cells from immunized mice were suspended in a-MEM tissue culture medium supplemented with 10 mM HEPES buffer, $5 \times 10^{-5}$ M 2-ME, antibiotics and 10% v/v fetal calf serum. $3 \times 10^7$ responder cells were cocultured with $1.5 \times 10^6$ syngeneic, HBcAg-expressing RBL5/C or HBsAg-expressing P815/S transfectants (irradiated with 20.000 rad) in 10 ml medium in upright 25 $cm^2$ tissue culture flasks in a humidified atmosphere/7% $CO_2$ at 37 °C. Cytotoxic effector populations were harvested after varying intervals of *in vitro* culture and washed twice. Serial dilutions of effector cells were cultured with $2 \times 10^3$ $^{51}$Cr-labeled targets in 200 μl round-bottom wells. Specific cytolytic activity of cells was tested in short-term $^{51}$Cr-release assays against transfected ($Ag^+$) or non-transfected ($Ag^-$) control targets. After a 4 h incubation at 37¡C, 100 μl of supernatant were collected for g-radiation counting. The percentage specific release was calculated as [(experimental release - spontaneous release)/(total release - spontaneous release)] x 100 . Total counts were measured by resuspending target cells. Spontaneously released counts were always less than 20 % of the total counts. Data shown represent mean specific lysis values of 5 mice ($\pm$ SD).

[2] *Determination of specifio serum antibody levels*. Antibodies against HBcAg were detected in mouse sera using the CORE$^R$ anti-HBc kit (cat.no. 2259-20; Abbott GmbH, Wiesbaden, FRG). Concentrations of anti-HBc were standardized against a reference standard of the Paul-Ehrlich-Institute (Langen, FRG). Anti-HBeAg antibody titers in sera of DNA-immunized mice were determined using the IMX anti-HBe assay (Abbot). This assay was also standardized using a reference from the Paul-Ehrlich-Institute.

**B3** Coexpression of antigen and a cytokine from a polycistronic construct in a single presenting cell enhances the cellular immune response

| # | mice immunized with plasmid | μg DNA/mouse | cytotoxic T response[1] | |
|---|---|---|---|---|
| | | | RBL5/C | RBL5 |
| 1 | pCMV-di/cAg/IL2 | 100 | 36 ± 11 | 3 ± 1 |
| 2 | pCMV-di/cAg/IL2 | 10 | 25 ± 9 | 7 ± 6 |
| 3 | pCMV-di/cAg/IL2 | 1 | 14 ± 10 | 4 ± 4 |
| 4 | pCMV-di/cAg/IL15 | 100 | 76 ± 21 | 6 ± 5 |
| 5 | pCMV-di/cAg/IL15 | 10 | 53 ± 35 | 4 ± 3 |
| 6 | pCMV-di/cAg/IL15 | 1 | 24 ± 11 | 3 ± 3 |

C57BL/6 (H-2$^b$) mice (5 animals per group) were immunized with plasmid DNA i.m. The cytotoxic T lymphocyte (CTL) response was read out 2 weeks post-immunization. All responses listed in the tables are of defined restriction and epitope specificity and mediated by CD4$^-$ CD8$^+$ T cells primed *in vivo*.

[1] *Cytotoxicity assay for specific T cell reactivity.* Spleen cells from immunized mice were suspended in a-MEM tissue culture medium supplemented with 10 mM HEPES buffer, 5 x 10$^{-5}$ M 2-ME, antibiotics and 10% v/v fetal calf serum. 3 x 10$^7$ responder cells were cocultured with 1.5 x 10$^6$ syngeneic, HBcAg-expressing RBL5/C or HBsAg-expressing P815/S transfectants (irradiated with 20.000 rad) in 10 ml medium in upright 25 cm$^2$ tissue culture flasks in a humidified atmosphere/7% CO$_2$ at 37 °C. Cytotoxic effector populations were harvested after varying intervals of *in vitro* culture and washed twice. Serial dilutions of effector cells were cultured with 2 x 10$^3$ $^{51}$Cr-labeled targets in 200 μl round-bottom wells. Specific cytolytic activity of cells was tested in short-term $^{51}$Cr-release assays against transfected (Ag$^+$) or non-transfected (Ag$^-$) control targets. After a 4 h incubation at 37¡C, 100 μl of supernatant were collected for g-radiation counting. The percentage specific release was calculated as [(experimental release - spontaneous release)/(total release - spontaneous release)] x 100 . Total counts were measured by resuspending target cells. Spontaneously released counts were always less than 20 % of the total counts. Data shown represent mean specific lysis values of 5 mice (± SD).

**References (DNA-based Vaccination to prime T cells)**

DNA immunization is a simple and efficient technique to sensitize T cells. It involves the injection into animals of expression plasmid DNA that encodes the antigen of interest (1-37). Transient expression of the DNA in an antigen-presenting cell (APC) in the skin (5,24), muscle (1,6,8-10,13,17,38), endothelium or vascular smooth muscle (3) allows the priming of immune responses. DNA immunization has been reported to successfully induce MHC class I-restricted CTL against different pathogens (17,25,27) including the core antigen of hepatitis C virus (31). DNA immunization has been shown to confer protection against influenza virus (13,17,26,30), rabies virus (27,35), lymphocytic choriomenigitis virus-induced pathology (32,37), malaria (25), and leishmaniasis (28,36).

1. **Jiao, S., P. Williams, R. K. Berg, B. A. Hodgeman, L. M. Liu, G. Repetto, and J. A. Wolff**. 1992. Direct gene transfer into nonhuman primate myofibers in vivo. *Hum. Gene Ther. 3*:21.

2. **Nabel, E. G., D. Gordon, Z. Y. Yang, L. Xu, H. San, G. E. Plautz, B. Y. Wu, X. Gao, L. Huang, and G. J. Nabel**. 1992. Gene transfer in vivo with DNA-liposome complexes: lack of auto-immunity and gonadal localization. *Hum. Gene Ther. 3*:649.

3. **Nabel, E. G., G. Plautz, and G. J. Nabel**. 1992. Transduction of a foreign histocompatibility gene into the arterial wall induces vasculitis. *Proc. Natl. Acad. Sci. U. S. A. 89*:5157.

4. **Stewart, M. J., G. E. Plautz, L. Del Buono, Z. Y. Yang, L. Xu, X. Gao, L. Huang, E. G. Nabel, and G. J. Nabel**. 1992. Gene transfer in vivo with DNA-liposome complexes: safety and acute toxicity in mice. *Hum. Gene Ther. 3*:267.

5. **Tang, D. C., M. DeVit, and S. A. Johnston**. 1992. Genetic immunization is a simple method for eliciting an immune response. *Nature 356*:152.

6. **Wolff, J. A., J. J. Ludtke, G. Acsadi, P. Williams, and A. Jani**. 1992. Long-term persistence of plasmid DNA and foreign gene expression in mouse muscle. *Hum. Mol. Genet. 1*:363.

7. **Wolff, J. A., M. E. Dowty, S. Jiao, G. Repetto, R. K. Berg, J. J. Ludtke, P. Williams, and D. B. Slautterback**. 1992. Expression of naked plasmids by cultured myotubes and entry of plasmids into T tubules and caveolae of mammalian skeletal muscle. *J. Cell Sci. 103*:1249.

8. **Davis, H. L., R. G. Whalen, and B. A. Demeneix**. 1993. Direct gene transfer into skeletal muscle in vivo: factors affecting efficiency of transfer and stability of expression. *Hum. Gene Ther. 4*:151.

9. **Davis, H. L., B. A. Demeneix, B. Quantin, J. Coulombe, and R. G. Whalen**. 1993. Plasmid DNA is superior to viral vectors for direct gene transfer into adult mouse skeletal muscle. *Hum. Gene Ther. 4*:733.

10. **Davis, H. L., M. L. Michel, and R. G. Whalen**. 1993. DNA-based immunization induces continuous secretion of hepatitis B surface antigen and high levels of circulating antibody. *Hum. Mol. Genet. 2*:1847.

11. **Duzgunes, N. and P. L. Felgner**. 1993. Intracellular delivery of nucleic acids and transcription factors by cationic liposomes. *Methods Enzymol. 221*:303.

12. **Fynan, E. F., R. G. Webster, D. H. Fuller, J. R. Haynes, J. C. Santoro, and H. L. Robinson**. 1993. DNA Vaccines: protective immunizations by parenteral, mucosal, and gene-gun inoculations. *Proc. Natl. Acad. Sci. U. S. A. 90*:11478.

13. **Montgomery, D. L., J. W. Shiver, K. R. Leander, H. C. Perry, A. Friedman, D. Martinez, J. B. Ulmer, J. J. Donnelly, and M. A. Liu**. 1993. Heterologous and homologous protection against influenza A by DNA vaccination: optimization of DNA vectors. *DNA Cell Biol. 12*:777.

14. **Nabel, E. G. and G. J. Nabel**. 1993. Direct gene transfer: basic studies and human therapies. *Thromb. Haemost. 70*:202.

15. **Nabel, G. J., E. G. Nabel, Z. Y. Yang, B. A. Fox, G. E. Plautz, X. Gao, L. Huang, S. Shu, D. Gordon, and A. E. Chang**. 1993. Direct gene transfer with DNA-liposome complexes in melanoma: expression, biologic activity, and lack of toxicity in humans. *Proc. Natl. Acad. Sci. U. S. A. 90*:11307.

16. **Plautz, G. E., Z. Y. Yang, B. Y. Wu, X. Gao, L. Huang, and G. J. Nabel**. 1993. Immunotherapy of malignancy by in vivo gene transfer into tumors [see comments]. *Proc. Natl. Acad. Sci. U. S. A. 90*:4645.

17. **Ulmer, J. B., J. J. Donnelly, S. E. Parker, G. H. Rhodes, P. L. Felgner, V. J. Dwarki, S. H. Gromkowski, R. R. Deck, C. M. DeWitt, A. Friedman, and et al**. 1993. Heterologous protection against influenza by injection of DNA encoding a viral protein [see comments]. *Science 259*:1745.

18. **Barry, M. A., M. E. Barry, and S. A. Johnston**. 1994. Production of monoclonal antibodies by genetic immunization. *Biotechniques 16*:616.

19. **Davis, H. L., M. L. Michel, M. Mancini, M. Schleef, and R. G. Whalen**. 1994. Direct gene transfer in skeletal muscle: plasmid DNA-based immunization against the hepatitis B virus surface antigen. *Vaccine 12*:1503.

20. **Felgner, J. H., R. Kumar, C. N. Sridhar, c. J. Wheeler, Y. J. Tsai, R. Border, P. Ramsey, M. Martin, and P. L. Felgner**. 1994. Enhanced gene delivery and mechanism studies with a novel series of cationic lipid formulations. *J. Biol. Chem. 269*:2550.

21. **Nabel, E. G., V. J. Pompili, G. E. Plautz, and G. J. Nabel**. 1994. Gene transfer and vascular disease. *Cardiovasc. Res. 28*:445.

22. **Nabel, G. J., A. E. Chang, E. G. Nabel, G. E. Plautz, W. Ensminger, B. A. Fox, P. Felgner, S. Shu, and K. Cho**. 1994. Immunotherapy for cancer by direct gene transfer into tumors. *Hum. Gene Ther. 5*:57.

23. **Plautz, G. E., E. G. Nabel, B. Fox, Z. Y. Yang, M. Jaffe, D. Gordon, A. Chang, and G. J. Nabel**. 1994. Direct gene transfer for the understanding and treatment of human disease. *Ann. N. Y. Acad. Sci. 716*:144.

24. **Raz, E., D. A. Carson, S. E. Parker, T. B. Parr, A. M. Abai, G. Aichinger, S. H. Gromkowski, M. Singh, D. Lew, M. A. Yankauckas, and et al**. 1994. Intradermal gene immunization: the possible role of DNA uptake in the induction of cellular immunity to viruses. *Proc. Natl. Acad. Sci. U. S. A. 91*:9519.

25. **Sedegah, M., R. Hedstrom, P. Hobart, and S. L. Hoffman**. 1994. Protection against malaria by immunization with plasmid DNA encoding circumsporozoite protein. *Proc. Natl. Acad. Sci. U. S. A. 91*:9866.

26. **Ulmer, J. B., R. R. Deck, C. M. DeWitt, A. Friedman, J. J. Donnelly, and M. A. Liu**. 1994. Protective immunity by intramuscular injection of low doses of influenza virus DNA vaccines. *Vaccine 12*:1541.

27. **Xiang, Z. Q., S. Spitalnik, M. Tran, W. H. Wunner, J. Cheng, and H. C. J. Ertl**. 1994. Vaccination with a plasmid vector carrying the rabies virus glycoprotein gene induces protective immunity against rabies virus. *Virology 199*:132.

28. **Xu, D. and F. Y. Liew**. 1994. Genetic vaccination against leishmaniasis. *Vaccine 12*:1534.

29. **Davis, H. L., R. Schirmbeck, J. Reimann, and R. G. Whalen**. 1995. DNA-mediated immunization in mice induces a potent MHC class I-restricted cytotoxic T lymphocyte response to Hepatitis B virus surface antigen. *Hum. Gene Ther. 6*:1447.

30. **Donnelly, J. J., A. Friedman, D. Martinez, D. L. Montgomery, J. W. Shiver, S. L. Motzel, J. B. Ulmer, and M. A. Liu**. 1995. Preclinical efficacy of a prototype DNA vaccine: enhanced protection against antigenic drift in influenza virus. *Nature-Medicine 1*:583.

31. **Lagging, L. M., K. Meyer, D. Hoft, M. Houghton, R. B. Belshe, and R. Ray**. 1995. Immune response to plasmid DNA encoding the hepatitis C virus core protein. *J. Virol. 69*:5859.

32. **Martins, L. P., L. L. Lau, M. S. Asano, and R. Ahmed**. 1995. DNA vaccination against persistent viral infection. *J. Virol. 69*:2574.

33. **Michel, M. L., H. L. Davis, M. Schleef, M. Mancini, P. Tiollais, and R. G. Whalen**. 1995. DNA-mediated immunization to the hepatitis B surface antigen in mice: aspects of the humoral response mimic hepatitis B viral infection in humans. *Proc. Natl. Acad. Sci. U. S. A. 92*:5307.

34. **Schirmbeck, R., W. Böhm, K. Ando, F. V. Chisari, and J. Reimann**. 1995. Nucleic acid vaccination primes hepatitis B surface antigen-specific cytotoxic T lymphocytes in nonresponder mice. *J. Virol. 69*:5929.

35. **Xiang, Z. Q. and H. C. J. Ertl**. 1995. Manipulation of the immune response to a plasmid-encoded viral antigen by coinoculation with plasmids expressing cytokines. *Immunity 2*:129.

36. **Xu, D. and F. Y. Liew**. 1995. Protection against leishmaniasis by injection of DNA encoding a major surface glycoprotein, gp63, of *L.major. Immunology 84*:173.

37. **Yokoyama, M., J. Zhang, and J. L. Whitton**. 1995. DNA immunization confers protection against lethal lymphocytic chorio-meningitis virus infection. *J. Virol. 69*:2684.

38. **Wang, B., K. E. Ugen, V. Srikantan, M. G. Agadjanyan, K. Dang, Y. Refaeli, A. I. Sato, J. Boyer, W. V. Williams, and D. B. Weiner**. 1993. Gene inoculation generates immune responses against human immunodeficiency virus type 1. *Proc. Natl. Acad. Sci. U. S. A. 90*:4156.

## Glossary

Ag+: antigen transfected
Ag-: antigen non-transfected
antigen presenting cell: cell presenting antigen by means of MHC
APC = antigen presenting cell

5-azacytidin: nucleotide analog

B cell: humoral system associated lymphocyte
BCMGneo: cf fig 1
BHK-21 cell: baby hamster kidney 21 cell
cAg = core antigen, especially HBV core antigen
CD4+: T cell subset
CD8+: T cell subset
cistron: DNA encoding a peptide, cf Lexikon Biochemie, 2nd ed (1981) 146
class I: cf MHC class I
class II: cf MHC class II
CMV = cytomegalo virus
CMVp = CMV promoter, commercially available
cre: cf Gossen & Bujard in PNAS USA, 89 (1992) 5547-5551
cT = Simian (papova) virus (mutant cytoplasmic) large tumor
cT-Ag: cT antigen
cytocidal: refers to cyto-suicidal
cytokine: kind of effector molecule; cf eg IFN, IL and GM-CSF
cytosolic proteins: proteins synthesized within a cell that are not associated with membranes during synthesis
CTL = cytotoxic T lymphocytes
DNA immunizing: cf eg WO-A1-9 011 092 = PCT/US 90/01 515
DOTAP: N-(1-(2,3-dioleoyloxy)-propyl)-N,N-trimethylammonium methylsulfate; liposome formulation for a cationic liposome-mediated tranfection of DNA into eukaryotic cells
effector function: eg effector molecule
effector molecule: eg cytokine
endocytosis: mechanism of uptake of exogenous material of a size of 10 to 100 nm by eukarytic cells
endogenous: refers to intracellular antigen and MHC class I restricted killer cell response
episomal: refers to plasmids integrateable into a host genom
ER = endoplasmic reticulum
exogenous: refers to extracellular antigen and MHC clas II restricted helper cell response
G418: neomycin derivative
ganciclovir: drug used for killing of suicide constructs
GM-CSF = granulocyte-macrophage colony stimulating factor (cytokine)
haplotype-specificity: binding of an antigenic peptide to MHC class I or II molecules is specific for the allelic variant of the respective MHC gene product
HBcAg = HBV core antigen
HBs: cf HBsAg
HBsAg: HBs antigen
HBV = hepatitis B virus
heavy chain/β2m microglobulin dimers: dimers formed by a heavy chain and β2m microglobulin
helper cell: refers to specific T lymphocyte; cf also killer cell
HPV = human papilloma virus
HSV = herpes simplex virus
HSVtk: fusion protein of HSV and tk
HTL = helper T lymphocyte
humoral system: refers to immune system in body fluids
hyg = hygromycin B phosphotransferase
hygtk: fusion protein of hyg and tk
IFNg = interferon gamma
IFN-gamma = interferon gamma
IgG light chain: refers to one chain type of an immunoglobuline class G
IgG heavy chain: refers to one chain type of an immunoglobuline class G
IL = interleucin such as IL-2, IL-4, IL-6, IL-7, IL-10, IL-12 and IL-15
IRES: polio virus derived element, cf WO-A1-9 405 785 page 10
isotype profil of antibodies: alternative forms of the constant region of antibody molecules that mediated different types of effector function
killer cell: refers to specific T lymphocyte; cf also helper cell
L1: HPV surface antigen
LS = HBV large surface antigen (S1/S2/sAg)
LTR = long terminal repeat of retrovirus

MHC = major histocompatibility complex glycoprotein, eg class I or class II

mu = murine

MPSV = myeloproliferative (retro) virus

mu-GM-CSF: cf fig 22 and 32

mu-IFNg: cf fig 23 and 33

mu-IL2: cf fig 18 and 26

mu-IL4: cf fig 19 and 27

mu-IL7: cf fig 20 and 28

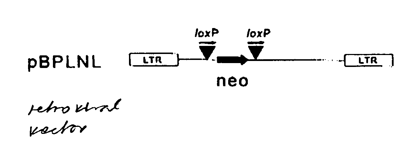

mu-IL12: cf fig 24, 25, 30 and 35

mu-IL15: cf fig 21 and 29

myocytes: differentiated muscle cell

neo: gene encoding neomycin resistance (confers resistance to toxic action of G418)

NIH3T3 cell: commercially available fibroblast line (often used for transfection studies)

pA: cf pBC-2 according to WO-A1-9 405 785

pBabePuro: cf Morgenstern & Land in Nucleic Acids Research, 18 (1990) 3587-3596

pBPLNL: pBabePuro with integrated loxP-neo-loxP cassette

pBR: refers to plasmid type commercially available

pCMV-1: cf fig 1-3

pCMV-2: cf fig 1-3

pCMV-3: cf fig 5-6

pCMV-8: promotor region from the immediate early region of HCMV

pCMV-di: cf fig 4, dicistronic vector according WO-A1-9 405 785

pCMV-di/cAg/sAg: cf fig 10

pCMV-di/L1/sAg: cf fig 16

pCMV-tri: cf fig 7

pCMV-tri/cAg/LS/pol: cf fig 14

pCMV-tri/L1/sAg/cTAg: cf fig 17

pol = polymerase

polyadenylation signal: used as termination signal

pSBC-1: expression plasmid according to WO-A1-9 405 785

pSBC-2: expression plasmid according to WO-A1-9 405 785

presenting cell: cf antigen-presenting cell

priming: making immunologically responsive by exposure to antigen

pTBC: cf Kischhoff et al in Trends Genet, 11 (1995) 219-220

pTCH: a derivative of pTBC

pUHD15-1: encoding TET-repressible transactivator TA puromycin acetyl transferase gene: used as puromycin resistance gene

release cytokine: activated T cells produce cytokines and secrete them into the extracellular medium

restricting molecule: refers to allele specific presentation of antigenic peptide

S1/S2/sAg = HBV large surface antigen

sAg = HBV small surface antigen

SEAP = secreted alcaline phosphatase

surface marker: a histotype-specific molecule expressed by a cell on the surface membrane that can be used to identify its developmental lineage

SV40 = Simian virus 40

SV40p = SV40 promoter

T cell: cell system associated lymphocyte

TA = transactivator (repressible by TET), cf Gossen & Bujard in PNAS USA, 89 (1992) 5547-5551

TAg: viral antigen, ie T antigen

TET = teracycline

TH1-type: subset of CD4+ T cells (that secrete IL-2 and interferon-gamma but not IL-4, 5, 6 and 10)

TH2-type: subset of CD4+ T cells (that secrete IL-4, 5, 6 and 10 but not interferon-gamma)

tk = thymidin kinase

transfection: cf Römpp Biotechnologie, (1992) 775

transfer: refers to DNA transfer

transscription: cf Römpp Biotechnologie, (1992): 777

wt = wild type

iC = °C

$\Gamma$ = R

Figure 2: **MOLECULE DEFINITION**

NAME:              pCMV-1
DATE:              04-MAR-1996
SIZE:              3849 bps DNA CIRCULAR
DESCRIPTION:       cloning see Andreas I, p.161
NOTES:
                   promoter exchange in pSBC-1:
                   XbaI, Klenow-treated and then SalI-cut hCMV-promoter carrying fragment from BCMGneo was
                   cloned into pSBC-1, ClaI-cut, Klenow-treated and then XhoI-cut
                   CMV-promoter, intron and multicloning region were sequenced by Peter Pudollek.
                   file: pCMV-1

Figure 3: **MOLECULE DEFINITION**

NAME:              pCMV-2
DATE:              04-MAR-1996
SIZE:              3226 bps DNA CIRCULAR
DESCRIPTION:       cloning see Andreas I, p.161
NOTES:
                   promoter exange in pSBC-2:
                   XbaI, Klenow-treated and then SalI-cut hCMV promoter carrying fragment from BCMGneo was
                   cloned into pSBC-2, ClaI-cut, Klenow-treated and then XhoI-cut
                   CMV-promoter, intron and multicloning region were sequenced by Peter Pudollek.

Figure 5: **MOLECULE DEFINITION**

NAME:              pCMV-3
DATE:              04-MAR-1996
SIZE:              3868 bps DNA CIRCULAR
DESCRIPTION:       protocoll see Birgit I, p.16
NOTES:
                   vector fusion between pCMV-1 and pCMV-2 via EcoRI/MstI to generate a vector-cassette for tricistronic expression
                   file: pcmv-3

Figure 8: **MOLECULE DEFINITION**

NAME:              pCMV-1/cAg
DATE:              26-OCT-1995
SIZE:              4413 bps DNA CIRCULAR
DESCRIPTION:       cloning and expresion see DorisI,p.70 and 90
NOTES:
                   XhoI-fragment from pBlue-c was cloned into SalI-digested pCMV-1
                   file: CMV1-C

Figure 9: **MOLECULE DEFINITION**

NAME:              pCMV-2/sAg
DATE:              06-MAR-1996
SIZE:              4081 bps DNA CIRCULAR
DESCRIPTION:       cloning see Peter Pudollek
NOTES:
                   EcoRI/HindIII-fragment from pCMV-1/sAg carrying HBV-sAg was cloned into EcoRI/HindIII-
                   digested pCMV-2
                   file: cmv2-sii

Figure 11: **MOLECULE DEFINITION**

NAME:          pCMV-2/LS
DATE:           06-MAR-1996
SIZE:           4544 bps DNA CIRCULAR
DESCRIPTION:  cloning see Peter Pudollek
NOTES:
                 BglII, Klenow-treated and then BstXI-digested s1s2-carrying fragment from HBV, strain AYW, was cloned into EcoRI, Klenow-treated and then BstXI-cut pCMV-2/sAg.
                 file: cmv2-ls

Figure 12: **MOLECULE DEFINITION**

NAME:          pCMV-3/pol
DATE:           06-MAR-1996
SIZE:           6378 bps DNA CIRCULAR
DESCRIPTION:
NOTES:
                 SalI-fragment from pBlue-pol was cloned into SalI-digested pCMV-3
                 file: cmv3-pol

Figure 13: **MOLECULE DEFINITION**

NAME:          pCMV-1/L1
DATE:           05-MAR-1996
SIZE:           5423 bps DNA CIRCULAR
DESCRIPTION:  cloning see Anja
NOTES:
                 XbaI, Klenow-treated and then HindIII-cut fragment from L1-blueII was cloned into SmaI/HindIII-digested pCMV-1
                 file: cmv1-l1

Figure 15: **MOLECULE DEFINITION**

NAME:          pCMV-3/cTAg
DATE:           05-MAR-1996
SIZE:           4947 bps DNA CIRCULAR
DESCRIPTION:
NOTES:
                 SalI-fragment from pBlue-cT was cloned into SalI-digested pCMV-3
                 file: cmv3-ct

Figure 18: **MOLECULE DEFINITION**

NAME:          pCMV-2/IL2
DATE:           26-OCT-1995
SIZE:           3793 bps DNA CIRCULAR
DESCRIPTION:  cloning and expression see Dorisl,p.83 and 94
NOTES:
                 XhoI-fragment from BMG-IL2 (provided by Karasuyama and Melchers, Basel, Switzerland) was cloned into SalI-digested pCMV-2
                 file: CMV2-IL2

Figure 19: **MOLECULE DEFINITION**

NAME:          pCMV-2/IL4
DATE:           26-OCT-1995
SIZE:           3739 bps DNA CIRCULAR
DESCRIPTION:  cloning and expression see Dorisl,p.84 and 95

NOTES:

Xhol-fragment from BCMG-IL4 (provided by Karasuyama and Melchers, Basel, Switzerland) was cloned into Sall-digested pCMV-2
file: CMV2-IL4

Figure 20: **MOLECULE DEFINITION**

NAME: pCMV-2/IL7
DATE: 04-MAR-1996
SIZE: 3690 bps DNA CIRCULAR
DESCRIPTION: cloning see BirgitI, p.18
NOTES:

IL-7-containing fragment was cut out from pIL7-CEH-A2 with SacI/HindIII and cloned into SacI/HindIII-cut p7,5K (provided by H.-J. Schlicht). IL-7 was exised from p7,5K-IL-7 with SmaI/HindIII and cloned into SmaI/HindIII-digested pCMV-2
file: cmv2-il7

Figure 21: **MOLECULE DEFINITION**

NAME: pCMV-2/IL15
DATE: 04-MAR-1996
SIZE: 3785 bps DNA CIRCULAR
DESCRIPTION: cloning see DorisI,p.67
NOTES:

EcoRI/Sall-fragment from pBlue-IL15 was cloned into EcoRI/Sall-digested pCMV-2
file: CMV-IL15

Figure 22: **MOLECULE DEFINITION**

NAME: pCMV-2/GMCSF
DATE: 06-MAR-1996
SIZE: 3670 bps DNA CIRCULAR
DESCRIPTION: cloning and expression see DorisI,p.85 and 95
NOTES:

BamHI-fragment from pBlue-GM/CSF was cloned into BamHI-site of pCMV-2
file: CMV2-GMC

Figure 24: **MOLECULE DEFINITION**

NAME: pCMV-2/msp40
DATE: 04-MAR-1996
SIZE: 4307 bps DNA CIRCULAR
DESCRIPTION: cloning see Birgit I, p.
NOTES:

SmaI/HindII-fragment from pBlue-msp40 was cloned into SmaI-digested pCMV-2
file: cmv2-m40

Figure 23: **MOLECULE DEFINITION**

NAME: pCMV-2/IFNg
DATE: 16-FEB-1996
SIZE: 3903 bps DNA CIRCULAR
DESCRIPTION: cloning see Birgit I, p.
NOTES:

VspI-fragment from murine IFNg-carrying plasmid pMS10 was Klenow-treated and cloned into SmaI-digested pCMV-2
file: cmv2-ifn

Figure 25: **MOLECULE DEFINITION**

NAME:               pCMV-3/msp35
DATE:               06-MAR-1996
SIZE:               4579 bps DNA CIRCULAR
DESCRIPTION:        cloning see Birgit I, p.
NOTES:

                    EcoRI/HindII-fragment from pBlue-msp35 was Klenow-treated abd cloned into SmaI-digested
                    pCMV-3
                    file: cmv3-m35

Figure 26: **MOLECULE DEFINITION**

NAME:               pCMV-cAg/IL2
DATE:               04-MAR-1996
SIZE:               5004 bps DNA CIRCULAR
DESCRIPTION:        cloning see Peter Pudollek
NOTES:
                    vector-fusion between pCMV-1/cAg and pCMV-2/IL2 via MstI/NotI-fragments resulting the bicistronic expression construct
                    file: c-il2

Figure 27: **MOLECULE DEFINITION**

NAME:               pCMV-c/IL4
DATE:               04-MAR-1996
SIZE:               4950 bps DNA CIRCULAR
DESCRIPTION:        cloning see Peter Pudollek
NOTES:
                    vector-fusion between pCMV-1/cAg and pCMV-2/IL4 via MstI/NotI-fragments resulting the bicistronic expression construct
                    file: c-il4

Figure 28: **MOLECULE DEFINITION**

NAME:               pCMV-c/IL7
DATE:               06-MAR-1996
SIZE:               4901 bps DNA CIRCULAR
DESCRIPTION:        cloning see Peter Pudollek
NOTES:
                    vector-fusion between pCMV-1/cAg and pCMV-2/IL7 via MstI/NotI-fragments resulting the bicistronic expression construct

Figure 29: **MOLECULE DEFINITION**

NAME:               pCMV-c/IL15
DATE:               06-MAR-1996
SIZE:               4996 bps DNA CIRCULAR
DESCRIPTION:        cloning see Peter Pudollek
NOTES:
                    vector-fusion between pCMV-1/cAg and pCMV-2/IL15 via MstI/NotI-fragments resulting the bicistronic expression construct
                    file: c-il15

Figure 30: **MOLECULE DEFINITION**

NAME:               pCMV-c/msp40
DATE:               04-MAR-1996
SIZE:               5518 bps DNA CIRCULAR

DESCRIPTION:    cloning see Birgit I, p.
NOTES:

        vector-fusion between pCMV-1/cAg and pCMV-2/IL12 msp40 via MstI/NotI-fragments resulting the bicistronic expression construct
        file: c-msp40

Figure 31. **MOLECULE DEFINITION**

NAME:           pCMV-c/IL12
DATE:           04-MAR-1996
SIZE:            6895 bps DNA CIRCULAR
DESCRIPTION:    cloning see Birgit I, p.
NOTES:

        NotI-fragment from pCMV-3/IL12 msp35 was cloned into NotI-digested pCMV-di/cAg/IL12 msp40 resulting in the tricistronc expression construct
        file: c-il12

Figure 32: **MOLECULE DEFINITION**

NAME:           pCMV-c/GMCSF
DATE:           04-MAR-1996
SIZE:            4881 bps DNA CIRCULAR
DESCRIPTION:    cloning see Peter Pudollek
NOTES:

        vector-fusion between pCMV-1/cAg and pCMV-2/GMCSF via MstI/NotI-fragments resulting the bicistronic expression construct
        file: c-gmcsf

Figure 33: **MOLECULE DEFINITION**

NAME:           pCMV-c/IFNg
DATE:           04-MAR-1996
SIZE:            5114 bps DNA CIRCULAR
DESCRIPTION:    cloning see Peter Pudollek
NOTES:

        vector-fusion between pCMV-1/cAg and pCMV-2/IFNg via XmnI/NotI-fragments resulting the bicistronic expression construct
        file: c-ifng

**Claims**

1. Polycistronic expression construct of a vector comprising at least one antigen gene and at least one cytokine gene.

2. Polycistronic expression construct of a vector comprising at least two antigen genes and, facultatively, at least one cytokine gene.

3. Expression construct according to claim 1 or 2, **characterized** in that the antigen genes are viral, bacterial, fungal, protozoal, tumor and/or prion antigens.

4. Expression construct according to any of the preceding claims, **characterized** in that the antigen genes are from the same and/or different pathogens.

5. Expression construct according to any of the preceding claims, **characterized** by one or more genes coding for chimeric proteins composed of immunogenic domains of different proteins.

6. Expression construct according to any of the preceding claims, **characterized** in that the antigen genes are derived from small viruses, especially pathogenic strains of hepadna, papova, and/or retro viruses.

7. Expression construct according to any of the preceding claims, **characterized** by HBcAg (cAg), large and/or small HBsAg (LS and/or sAg), polymerase (pol) of hepatitis B virus (HBV), surface antigen L1 of papilloma virus and/or large nucleoprotein T-Ag of SV 40; as viral antigens.

8. Expression construct according to any of the preceding claims, **characterized** by cytokine genes encoding IL-2, IL-4, IL-6, IL-7, IL-10, IL-12 and/or IL-15; IFN, especially IFNalpha, IFNβ and/or IFNg; and/or GM-CSF.

9. Expression construct according to any of the preceding claims, **characterized** in that it comprises more than one and especially two or more than two genes of the same cytokine.

10. Expression construct according to any of the preceding claims, comprising the following genes:

cAg/sAg (Fig. 10),
L1/sAg (Fig. 16),
cAg/mu-IL2 (Fig. 26),
cAg/mu-IL4 (Fig. 27),
cAg/mu-IL7 (Fig. 28),
cAg/mu-IL15 (Fig. 29),
cAg/mu-GM-CSF (Fig. 32),
cAg/mu-IFNg (Fig. 33),
sAg/mu-IL2,
sAg/mu-IL4,
sAg/mu-IL7,
sAg/mu-IL15,
sAg/mu-GM-CSF,
sAg/mu-IFNg,
SV40cT-Ag/mu-L2,
SV40cT-Ag/mu-L4,
SV40cT-Ag/mu-IL7,
SV40cT-Ag/mu-IL15,
SV40cT-Ag/mu-GM-CSF, and
SV40cT-Ag/mu-IFNg.

11. Expression construct according to any of claims 1 to 9, comprising the following genes:

cAg/LS/pol (Fig. 14),
L1/sAg/cTAg (Fig. 17),
cAg/mu-IL12/mu-IL12,
sAg/mu-IL12/mu-IL12, and
SV40cT-Ag/mu-IL12/mu-IL12.

12. Expression construct according to any of the preceding claims, **characterized** in that the vector is derived from pSPC-1 or pSPC-2.

13. Expression construct according to claim 1 to 9, **characterized** in that the CMVp sequence has been replaced for the SV40p sequence.

14. Expression construct according to any of the preceding claims, **characterized** by a suicide gene inducible with a therapeutically acceptable drug.

15. Expression construct according to claim 14, **characterized** by a suicide gene inducible with Ganciclovir.

16. Vaccine comprising an expression construct according to any of the preceding claims.

17. Use of an expression construct according to any of claims 1 to 15 for the production of a vaccine.

A1                                    figure 1

SspI
XmnI
ClaI
SV40p
t-intron
AmpR
pSBC-1
3470 bps
IRES
ori
pA
XhoI
EcoRI
SmaI
SalI
PstI
HindIII
KpnI
NotI
XmaIII

SspI
XmnI
ClaI
SV40p
t-intron
AmpR pSBC-2
2846 bps
ori
pA
XhoI
NotI
XmaIII
EcoRI
SmaI
BamHI
SalI
PstI
HindIII

XbaI
SalI
HindIII
CMVp
intron
poly A
XhoI
BamHI
NotI
BCMGneo
14335 bps
HindIII

pSBC-1 [ClaI,Klenow/XhoI] fragm.          CMV-promoter
or pSBC-2 [ClaI,Klenow/XhoI]-fragm.       BCMGneo [XbaI,Klenow/SalI]-fragm.

SspI
XmnI
CMVp
AmpR
MstI
ori
pCMV-1
3849 bps
t-intron
IRES
SV40 pA
NotI
XmaIII
EcoRI
SmaI
AvaI
SalI
PstI
HindIII

SspI
XmnI
CMVp
AmpR
MstI
ori
pCMV-2
3226 bps
t-intron
SV40 pA
NotI
XmaII
EcoRI
SmaI
AvaI
BamH
SalI
PstI
HindI

HA1                    figure 2

A1

figure 3

pCMV-2
3226 bps

2991, SspI
2784, XmnI
2409, MstI
3000
CMVp
500
AmpR
2500
ori
int
2000
1000
pA
1500

NotI, 818
XmaIII, 819
EcoRI, 826
SmaI, 831
AvaI, 831
BamHI, 836
SalI, 842
PstI, 848
HindIII, 856

A1

figure 4

pCMV-1
3849 bps

SspI
XmnI
MstI
AmpR
ori
CMVp
int
IRES
pA
EcoRI
SmaI
AvaI
SalI
PstI
HindIII
NotI
XmaIII

pCMV-2
3226 bps

SspI
XmnI
MstI
AmpR
ori
CMVp
int
pA
NotI
XmaIII
EcoRI
SmaI
AvaI
BamHI
SalI
PstI
HindIII

pCMV-1 [MstI or XmnI/NotI]     pCMV-2 [MstI or XmnI/NotI]
1. gene fragm.                 2. gene fragm.

pCMV-di
3873 bps

SspI
XmnI
MstI
AmpR
ori
CMVp
int
IRES
pA
EcoRI
AvaI
SmaI
BamHI
SalI
PstI
HindIII
BamHI
NotI
EcoRI
AvaI
SmaI
BamHI
SalI
PstI
HindIII

A1

figure 5

A1         figure 6

A1

figure 7

A21

figure 8

4402,SmaI
4397,EcoRI

BglII,92
XbaI,98

XbaI,249

BglII,531
PstI,570
HindIII,576

int

cAg

CMVp

4000

IRES

3355,SspI

pCMV-1/cAg

1000

4413 bps

3148,XmnI

3000

AmpR

NotI,1206
XmaIII,1207

pA

2000

ori

2773,MstI

A2.1

Figure 9

4070,SmaI
4070,AvaI
4065,EcoRI
4058,XmaIII
4057,NotI

XbaI,120

int

CMVp

sAg

4000

Xbal,851
SaII,857
PstI,863
HindIII,869

**pCMV-2/sAg**

4081 bps

1000 — pA

3000

3004,SspI

2000

2797,XmnI

AmpR

ori

2422,MstI

A2.1                                    Figure 10

Plasmid map: pCMV-di/cAg/sAg, 5292 bps

Restriction sites and features:
- SmaI, AvaI, EcoRI
- BglII, XbaI
- XbaI
- BglII, AvaI, PstI, HindIII
- int, cAg
- CMV_R
- 5000
- IRES
- 1000
- NotI, EcoRI, AvaI, SmaI
- XbaI
- sAg
- 2000
- SspI
- XmnI
- AmpR, 4000
- ori
- MstI
- 3000
- pA
- XbaI, SalI, PstI, HindIII

A2.1

Figure 11

4537,EcoRI
4530,XmaIII
4529,NotI

EcoRI,343

int   s1

CMVp

s2

4000

sAg

3476,SspI

pCMV-2/LS

4544 bps

1000

3269,XmnI

AmpR   3000

pA

SalI,1329
PstI,1335
HindIII,1341

2000

2894,MstI

ori

A2.1

figure 12

6378,SalI
6367,SmaI
6362,EcoRI
6355,XmaIII
6354,NotI
6184,SacI

int

EcoRI,884

CMVp

6000

1000

pol

pCMV-3/pol

6378 bps

AmpR   5000

4719,MstI

ori

2000

4000

3000

IRES

pA

SalI,2510
PstI,2516
HindIII,2522

NotI,3152
XmaIII,3153

A2.1

figure 13

pCMV-1/L1

5423 bps

5404,EcoRI
Sspl,185
BamHI,522
Sspl,923
PstI,1159
EcoRI,1190
PstI,1375
SmaI,1559
XmaI,1559
PstI,1565
EcoRI,1571
HindIII,1583
BamHI,1805
NotI,2213
XmaIII,2214
4362,Sspl
4155,Xmnl
3780,Mstl
CMVp
int
L1
IRES
pA
AmpR
ori

A2.1

figure 14

from p CMV-1/cAg

SmaI
EcoRI

PstI
HindIII

NotI
EcoRI
SmaI
SalI

CMVp    int cAg

IRES

MstI

AmpR

ori

EcoRI

**pCMV-tri/cAg/s1s2sAg/pol**

8931 bps

pol

pA

sAg

HindIII
PstI
SalI

s2   s1   IRES

EcoRI

NotI
EcoRI

SalI
PstI
HindIII

A2.1

figure 15

4947,SalI
4941,BamHI
4936,SmaI
4936,AvaI
4931,EcoRI
4924,XmaIII
4923,NotI
4753,SacI

AvaI,5
XhoI,5
SspI,86

int

CMVp | cT

SspI,769

SspI,922
HindIII,1047
SalI,1079
PstI,1085
HindIII,1091

3870,SspI

4000

pCMV-3/cTAg 1000

4947 bps

1000

3663,XmnI

AmpR

IRES

BamHI,1313

3000 2000

3288,MstI

ori

pA

NotI,1721
XmaIII,1722

A2.2

figure 16

*pCMV-di/L1/sAg*

6302 bps

A2.2                                    figure 17

A3.1

figure 18

3787,BamHI
3782,SmaI
3782,AvaI
3777,EcoRI
3770,XmaIII
3769,NotI
3599,SacI

SphI,16
PvuII,156
SacI,177
BglII,272
HindIII,338

int
mu-IL2

PstI,573
HindIII,581

CMVp

3500

500

pA

pCMV-2/IL2

3793 bps

3000

1000

2716,SspI

2500

1500

AmpR

2000

2509,XmnI

ori

2134,MstI

A3.1        figure 19

3733,BamHI
3728,SmaI
3728,AvaI
3723,EcoRI
3716,XmaIII
3715,NotI
3545,SacI

SacI,279
PstI,375
ClaI,413
XmnI,455
PstI,519
HindIII,527

int

mu-IL4

CMVp

3500

500

pA

3000

**pCMV-2/IL4**

**3739 bps**

1000

2662,SspI

2500

1500

AmpR

2000

2455,XmnI

ori

2080,MstI

A3.1

figure 20

A3.1

figure 21

A3.1

figure 22

3670,BamHI
3665,SmaI
3665,AvaI
3660,EcoRI
3653,XmaIII
3652,NotI        PstI,21

Xmnl,233

BamHI,444
SalI,450
PstI,456
HindIII,464

int
mu-GMCSF

CMVp

3500

pA

500

3000

pCMV-2/GMCSF

3670 bps        1000

2500

1500

2599,SspI

2000

AmpR

2392,Xmnl

ori

2017,MstI

A3.1

figure 23

A3.1

figure 24

HindIII
EcoRI
XmaIII
NotI : PstI

AvaI
PstI

int

CMVp \
4000

IL12 msp40

**pCMV-2/IL12 msp40**

4307 bps

1000

pA

PstI
EcoRI
PstI
AvaI
SmaI
BamHI
SalI
PstI
HindIII

SspI

3000

XmnI

AmpR

2000

ori

MstI

A3.1                              figure 25

A3.2                                    figure 26

BamHI
SmaI
AvaI
EcoRI

                                                    AvaI
                                                    PstI
                                                    HindIII

         CMVp        int     cAg              BamHI

SspI                                          NotI
                                              EcoRI
                    pCMV-di/cAg/IL2           AvaI
XmnI       AmpR                               SmaI
                                              BamHI
                    5004 bps
                                  IRES
                                              mu-IL2

         ori
MstI                            pA            HindIII

                                              PstI
                                              HindIII

A3.2   figure 27

pCMV-di/cAg/IL4
4950 bps

A3.2  figure 28

pCMV-di/cAg/IL7

4901 bps

A3.2   figure 29

pCMV-di/cAg/IL15

4996 bps

A3.2  figure 30

pCMV-di/cAg/IL12 msp40
5518 bps

A3.2

figure 31

A3.2

*figure* 32

BamHI
SmaI
AvaI
EcoRI

AvaI
PstI
HindIII

int

cAg

CMVp

BamHI

SspI

IRES

NotI
EcoRI
AvaI
SmaI
BamHI
PstI

**pCMV-di/cAg/GMCSF**

4881 bps

XmnI

AmpR

mu-GMCSF

XmnI

ori

pA

MstI

BamHI
SalI
PstI
HindIII

A3.2

figure 33

Purpose: To isolate cells in which the first cistron (e.g. the cre recombinase) is under strict regulation of the tetracyclin-repressible promoter.

Problem: Many transfectants (cell clones having received the genes) show a constitutive base level (in presence of tet) and/or little inducibility (tet -)

# 1. Cotransfect pTCH and pUHD15-1

from pTCH the bicistronic construct, from pUHD15-1 the transactivator is expressed

## First selection for cells expressing pTCH and pUHD15-1

2 · Selection for Hygromycin resistance

## in absence of TET and Ganciclovir

active transactivator

pTCH

Polio-5'-UTR

cre    HygTk

tTA promoter

bicistronic mRNA ●~~~~~~~~~~~~~~AAAA

cre

HygTk

Hygromycin-resistant cells must express the bicistronic mRNA (waved line) because the transactivator is not repressed by tet. This indicates that the hygro-TK fusion protein and the also the cre protein must be expressed. The cells can survive the TK -activity since Ganciclovir is not in the cell culture medium.

Cells which do not express the bicistronic mRNA sufficiently, will not survive in the presence of Hygromycin. They must also express the gene of interest (cre) sufficiently.

## The same cells (see above) are now screened a second time

## 3. Select for Ganciclovir resistance

## in presence of TET

TET blocked transactivator

cre    HygTk

Due to the repression of the Tetracyclin-repressible promoter in the presence of tet, no bicistronic mRNA and no proteins encoded by this mRNA are made. The cells are resistant to Ganciclovir because the TK-activity from the HygTk fusion protein is absent.

Cells that show a base level expression of the bicistronic mRNA will produce HygTk fusion protein and incorporate Ganciclovir into their DNA. These cells will be killed.

EP 0 803 573 A1

pBPLNL

*loxP*     *loxP*

| LTR |          | LTR |

**neo**

*retro viral*
*vector*

European Patent Office

**EUROPEAN SEARCH REPORT**

Application Number

EP 96 10 6531

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X,D | GENETIC ENGINEERING NEWS, 1 March 1996, pages 14-16, XP002012721 DIFMOND P.F: "Therion combines technologies to produce vaccines and immunotherapeutics" | 1-6,16, 17 | C12N15/62 C07K14/005 C07K14/195 C07K14/37 C07K14/44 C07K14/47 C07K14/52 A61K39/295 |
| Y | * the whole document * | 9,14,15 | |
| X | EP-A-0 406 857 (TAKEDA CHEMICAL INDUSTRIES LTD) 9 January 1991 * page 4, line 50 - page 5, line 3; claims 1-35 * | 1,3-6, 16,17 | |
| X | US-A-4 859 465 (RUTTER WILLIAM J) 22 August 1989 * claims 1-4; example 2 * | 2-7,10 | |
| Y | EP-A-0 154 576 (INTERFERON SCIENCES INC) 11 September 1985 * abstract; claims 1,2,6 * | 9 | |
| Y | WO-A-94 13824 (UNIV PARIS CURIE ;KLATZMANN DAVID (FR); CARUSO MANUEL (FR)) 23 June 1994 * claims 1-7 * | 14,15 | **TECHNICAL FIELDS SEARCHED** (Int.Cl.6) C07K C12N |

The present search report has been drawn up for all claims

| Place of search | Date of completion of the search | Examiner |
|---|---|---|
| THE HAGUE | 5 September 1996 | Gurdjian, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)